# EUROPEAN PATENT SPECIFICATION

(11) **EP 3 818 951 B1**
(45) Date of publication and mention of the grant of the patent: **12.03.2025**
(21) Application number: 19844299.8
(22) Date of filing: 30.07.2019
(51) Int. Cl.: A61B 17/135, A61H 9/00

(54) **HEMOSTATIC INSTRUMENT**
HÄMOSTATISCHES INSTRUMENT
INSTRUMENT HÉMOSTATIQUE

(30) Priority: 01.08.2018 JP 2018145366
(43) Date of publication of application: 12.05.2021
(73) Proprietor: TERUMO Kabushiki Kaisha, Tokyo 151-0072 (JP)
(72) Inventor: WATANABE, Fumi, Kanagawa 259-0151 (JP); MIYASHITA, Masako, Fujinomiya-shi, Shizuoka 418-0015 (JP); HIOKI, Yuichi, Fujinomiya-shi, Shizuoka 418-0015 (JP); OKAMURA, Ryo, Kanagawa 259-0151 (JP); WADA, Satoshi, Fujinomiya-shi, Shizuoka 418-0015 (JP)
(74) Representative: Casalonga
(86) International application number: PCT/JP2019/029820
(87) International publication number: WO 2020/027123

(56) References cited:
- WO-A1-2008/126963
- WO-A1-2018/181314
- CN-A- 106 963 445
- JP-A- 2010 131 296
- KR-A- 20070 055 754
- US-A- 5 997 495
- US-A1- 2003 139 255
- US-A1- 2017 042 552
- US-A1- 2019 133 602

## Description

### Technical Field

The present invention relates to a hemostatic device.

### Background Art

As a catheter procedure, a procedure is known in which a blood vessel (for example, radial artery) in an arm of a patient is punctured, and various medical elongated bodies are introduced into the blood vessel via a puncture site formed in the blood vessel in the arm of the patient so as to perform treatment or therapy on a lesion area (see PTL 1 below). The catheter procedure utilizing the radial artery is referred to as transradial artery approach and is considered as a useful technique for, for example, coronary artery access and lower limb artery access.

A radial artery located in an arm of a human body is connected to a palmar artery which bypasses a hand side. Therefore, currently, as a new method of transradial artery approach, a catheter procedure using distal transradial approach (dTRA) has been attempted to access the palmar artery (including a distal radial artery) from an anatomical snuffbox located on a dorsal side of the hand or from a position around the snuffbox, and perform treatment through the vascular access site.

### Citation List

### Patent Literature

PTL 1: JP-A-2008-119517
US5997495A
US2003139255A1
KR20070055754A

### Summary of Invention

### Technical Problem

Blood vessels such as palmar arteries located in the hand are located in places where there are many movable parts such as fingers. For this reason, a shape around the puncture site in the hand changes with movement of the hand. Therefore, when hemostasis is performed on the puncture site, a pressing member placed on the hand is preferably an inflatable member that follows the movement of the hand and can easily adjust a compressive force on the puncture site.

However, when the pressing member is the inflatable member, the inflatable member exerts a force to inflate from the inside to the outside of the inflatable member in an inflated state. For this reason, in a hemostatic device having the inflatable member, a shape around a hemostatic site changes due to the movement of the hand, the inflatable member is shifted from the puncture site, and it may not be possible to properly maintain the compressive force of the inflatable member on the puncture site in some cases. Therefore, when hemostasis is performed on the puncture site of the hand, the hemostatic device having the inflatable member needs to suppress position shift of the inflatable member by force acting in a direction away from the puncture site, thereby appropriately securing the inflatable member to the puncture site. In this way, it is considered that the hemostatic device having the inflatable member can appropriately maintain the compressive force of the inflatable member on the puncture site even when the shape around the puncture site is changed by the movement of the hand.

In view of the above problems, an object of the invention is to provide a hemostatic device capable of suppressing position shift of an inflatable member with respect to a site where bleeding is to be stopped on a hand and appropriately maintaining a compressive force of the inflatable member on the site where bleeding is to be stopped on the hand even when a patient moves the hand while the inflatable member is inflated.

### Solution to Problem

The invention is defined by appended claim 1.

### Advantageous Effects of Invention

In the hemostatic device according to the aspect of the invention, the main body in which the inflatable member is disposed can be secured to the limb by disposing a part of the second arm portion between adjacent fingers of the patient and connecting the convex portion and the second arm portion while wrapping the first arm portion and the second arm portion around the limb of the patient. The first arm portion and the second arm portion are fastened to the limb by connecting the convex portion and the second arm portion, and the main body is secured to the limb. Further, by disposing the second arm portion between the adjacent fingers of the patient, it is possible to suppress position shift of the inflatable member disposed in the main body with respect to the site where bleeding is to be stopped. Further, the hemostatic device has a simple securing structure capable of tightening the inflatable member to the hand of the patient by connecting the two arm portions protruding from the main body. For this reason, the hemostatic device can reduce the number of arm portions installed for securing the inflatable member. In this way, the hemostatic device can suppress an increase in arm portions that may interfere with a medical device such as an introducer in a state where the hemostatic device is attached to the hand of the patient, and thus it is possible to easily remove the medical device from the site where bleeding is to be stopped even after the hemostatic device is attached. In addition, a movable range of the hand increases from a wrist side to a fingertip side of the hand. In the hemostatic device, the first arm portion and the second arm portion extend from the fingertip side to the wrist side of the hand while forming an obtuse angle, so that the first arm portion and the second arm portion can be connected on the wrist side of the hand of the patient. Therefore, in the hemostatic device, the first arm portion and the second arm portion can be secured on the wrist side where the movable range is small, and thus the main body can be secured to the limb while maintaining the movable range on the fingertip side of the hand. Note that an outer circumference of the hand becomes larger from the wrist side to the fingertip side of the hand in a state where the hand is spread. Therefore, since the first arm portion and the second arm portion extend in a direction opposite to a direction in which the outer circumference of the hand becomes larger, the first arm portion and the second arm portion can be reliably connected on the wrist side of the hand in the state where the hemostatic device is attached. Furthermore, in the hemostatic device, even in the case where the patient moves the hand with the inflatable member inflated, when the first arm portion and the second arm portion are secured in a state of being tightened to the limb of the patient, it is possible to prevent rising of the distal side (fingertip side) of the main body to which the inflatable member is connected, and to appropriately maintain the compressive force of the inflatable member on the site where bleeding is to be stopped formed on the dorsal side of the hand of the patient.

In the hemostatic device according to the other aspect of the invention, since the first arm portion and the second arm portion protrude from the main body so as to form an obtuse angle, when the hemostatic device is attached to the limb of the patient, the respective arm portions can be connected so that the entire finger of the patient is not covered by the covering member. For this reason, the patient is less likely to be restricted in movement of the hand even when the hemostatic device is attached, and thus a degree of freedom on the fingertip side can be increased. Further, since the hemostatic device has the deformable auxiliary member located on the distal side of the first inflatable portion, when the first inflatable portion is inflated, the auxiliary member presses the first inflatable portion against the limb of the patient to inhibit the first inflatable portion from rising in a direction away from the puncture site on the hand of the patient. As a result, the hemostatic device can maintain an appropriate compressive force on the site where bleeding is to be stopped while increasing the degree of freedom on the fingertip side. Furthermore, in the hemostatic device, since the first arm portion and the second arm portion extend from the fingertip side to the wrist side of the hand while forming an obtuse angle, the first arm portion and the second arm portion can be secured on the wrist side where the movable range is small. Therefore, the main body can be appropriately secured to the limb while maintaining the movable range on the fingertip side of the hand. Further, in the hemostatic device, since the first inflatable portion can be secured to the site where bleeding is to be stopped by the two arm portions including the first arm portion and the second arm portion and the auxiliary member, it is unnecessary to add different arm portions between the first arm portion and the second arm portion. Therefore, the operator, etc. can easily remove the medical device such as an introducer sheath after attaching the hemostatic device. Further, since the first arm portion and the second arm portion are connected in a state of being wrapped around the limb of the patient, the first arm portion and the second arm portion are pressed against the hand of the patient while pulling the main body located therebetween toward the both side portion sides of the main body. Since the auxiliary member is located between the first arm portion and the second arm portion, the inflatable member can be reliably pressed against the body surface of the hand of the patient by connection of the first arm portion and the second arm portion. Therefore, the hemostatic device can press the first inflatable portion against the limb of the patient by the auxiliary member, and reliably inhibit the first inflatable portion from rising in the direction away from the site where bleeding is to be stopped on the hand of the patient. In this way, the hemostatic device can reliably press the first inflatable portion against the hand of the patient and maintain an appropriate compressive force on the puncture site while increasing the degree of freedom on the fingertip side during attachment of the hemostatic device.

### Brief Description of Drawings

[Fig. 1] Fig. 1 is a diagram illustrating a hemostatic device according to a first embodiment, and is a plan view seen from an outer surface side of a main body of a covering member.
[Fig. 2] Fig. 2 is a diagram illustrating the hemostatic device according to the first embodiment, and is a plan view seen from an inner surface side of the main body of the covering member.
[Fig. 3] Fig. 3 is an enlarged view of a part of the hemostatic device.
[Fig. 4] Fig. 4 is a cross-sectional view of the hemostatic device taken along arrow 4-4 illustrated in Fig. 3, and a diagram illustrating a state in which an inflatable member inflates.
[Fig. 5A] Fig. 5A is a diagram schematically illustrating a usage example of the hemostatic device according to the first embodiment.
[Fig. 5B] Fig. 5B is a diagram schematically illustrating a usage example of the hemostatic device according to the first embodiment.
[Fig. 5C] Fig. 5C is a diagram schematically illustrating a usage example of the hemostatic device according to the first embodiment.
[Fig. 5D] Fig. 5D is a diagram schematically illustrating a usage example of the hemostatic device according to the first embodiment.
[Fig. 5E] Fig. 5E is a diagram schematically illustrating a usage example of the hemostatic device according to the first embodiment.
[Fig. 6] Fig. 6 is a diagram schematically illustrating a part of a cross section taken along arrow 6-6 illustrated in Fig. 5E.
[Fig. 7A] Fig. 7A is a diagram schematically illustrating another usage example of the hemostatic device according to the first embodiment.
[Fig. 7B] Fig. 7B is a diagram schematically illustrating another usage example of the hemostatic device according to the first embodiment.
[Fig. 7C] Fig. 7C is a diagram schematically illustrating another usage example of the hemostatic device according to the first embodiment.
[Fig. 7D] Fig. 7D is a diagram schematically illustrating another usage example of the hemostatic device according to the first embodiment.
[Fig. 8] Fig. 8 is a plan view of the hemostatic device according to a modification of the first embodiment.
[Fig. 9] Fig. 9 is a cross-sectional view of the hemostatic device taken along arrow 9-9 illustrated in Fig. 8.
[Fig. 10] Fig. 10 is a diagram illustrating a hemostatic device according to a second embodiment, and is a plan view seen from an outer surface side of a main body of a covering member.
[Fig. 11] Fig. 11 is a diagram illustrating the hemostatic device according to the second embodiment, and is a plan view seen from an inner surface side of the main body of the covering member.
[Fig. 12A] Fig. 12A is a diagram schematically illustrating a usage example of the hemostatic device according to the second embodiment.
[Fig. 12B] Fig. 12B is a diagram schematically illustrating a usage example of the hemostatic device according to the second embodiment.

### Description of Embodiments

Hereinafter, embodiments of the invention will be described with reference to the accompanying drawings. Note that the following description does not limit the technical scope or the meaning of terms described in the appended claims. In addition, the dimensions or scales on the drawings may be exaggerated for convenience of description, and different from actuality ones.

### (First embodiment)

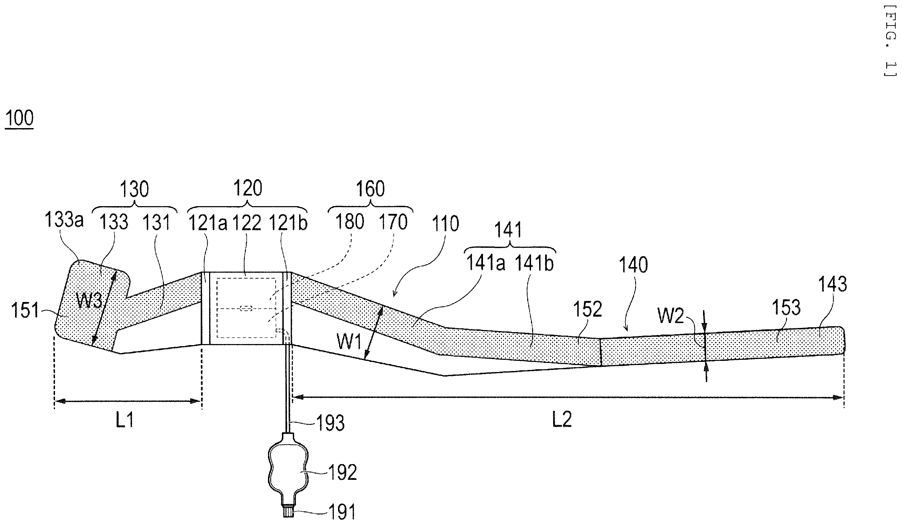

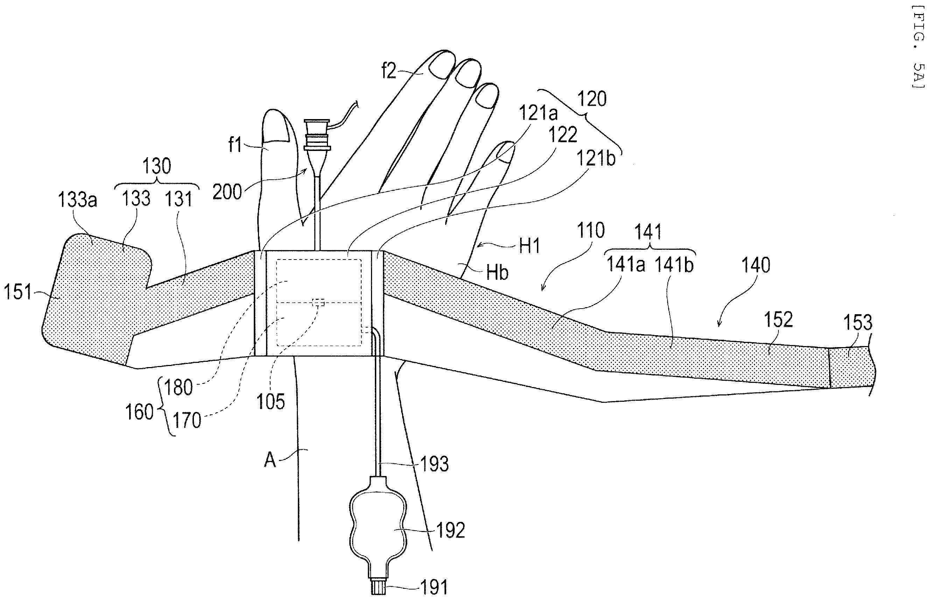

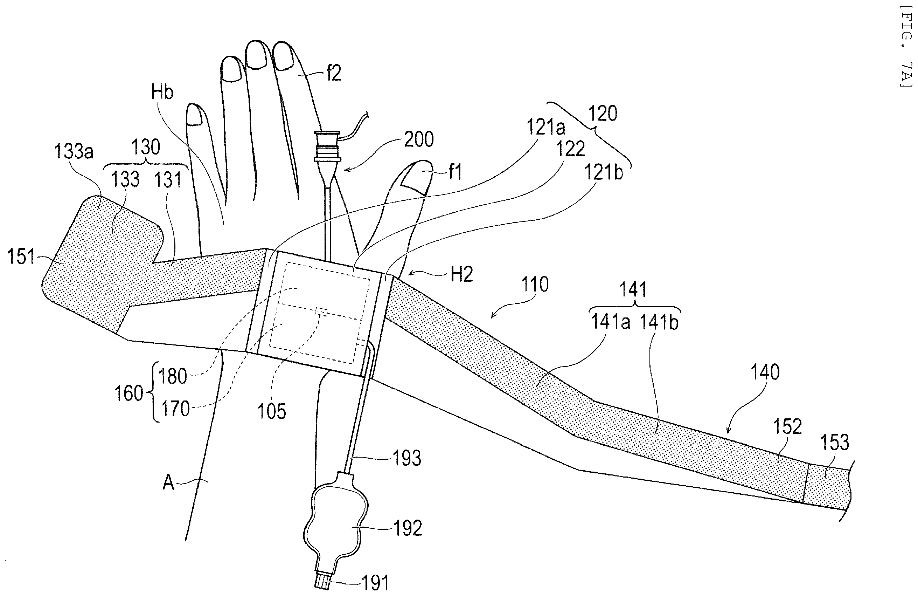

Figs. 1 to 4 are diagrams for description of a hemostatic device 100 according to a first embodiment, Figs. 5A to 6 are diagrams for description of usage examples of the hemostatic device 100, and Figs. 7A to 7D are diagrams for description of other usage examples of the hemostatic device 100.

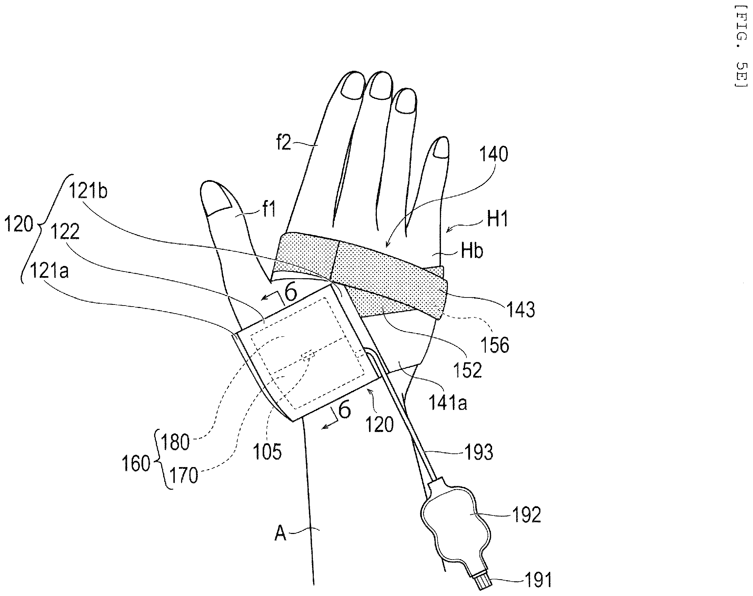

For example, as illustrated in Figs. 5E, 6, and 7D, when removing a sheath tube of an introducer 200 indwelling at a puncture site t1 (corresponding to a site where bleeding is to be stopped) formed on a radial artery side (for example, an artery around an anatomical snuffbox or a distal radial artery running on a fingertip side of the snuffbox) of a palmar artery (deep palmar artery) B1 running on a dorsal side Hb of a right hand H1 (or a left hand H2) located on the fingertip side of a forearm A of a patient, the hemostatic device 100 is used to perform hemostasis on the puncture site t1. Note that the anatomical snuffbox is a cavity in the hand located on the radial side of the forearm A when the patient spreads a thumb f1 of the right hand H1 or the left hand H2.

In brief, as illustrated in Figs. 1, 2, and 6, the hemostatic device 100 includes a covering member 110 configured to be disposed to cover the puncture site t1 on the right hand H1 of the patient, a plurality of securing members 151, 152, 153, 154, 155, and 156 for securing the covering member 110 while the covering member 110 covers the puncture site t1, and an inflatable member 160 connected to the covering member 110 and configured to be inflated by injection of a fluid.

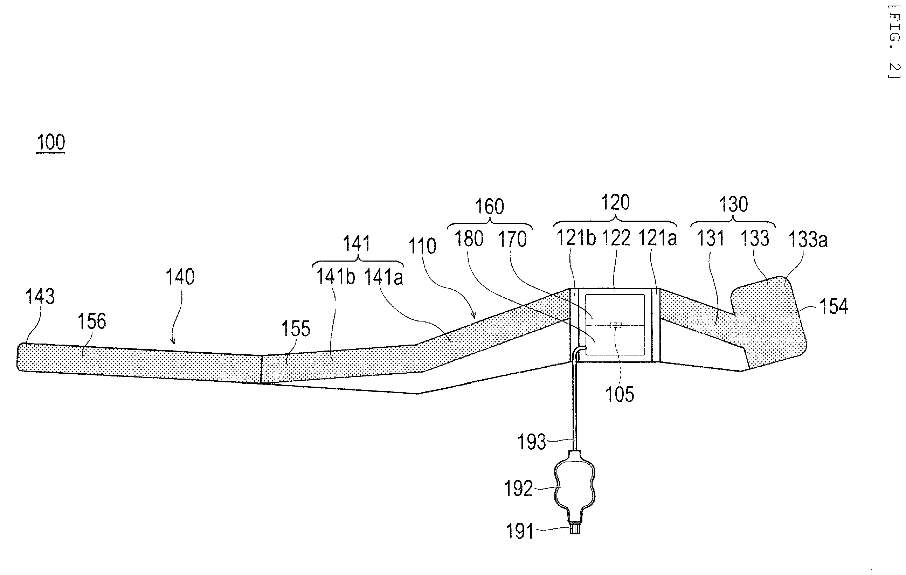

Fig. 1 illustrates a plan view of the hemostatic device 100 seen from an outer surface side of a main body 120 of the covering member 110, and Fig. 2 illustrates a plan view of the hemostatic device 100 seen from an inner surface side of the main body 120 of the covering member 110. The inner surface of the main body 120 is a surface on a side to which the inflatable member 160 disposed to face a body surface of the patient when the hemostatic device 100 is attached to the patient is connected, and the outer surface of the main body 120 is a surface opposite to the inner surface (surface on a side where a support member 125 is disposed in the present embodiment) . In addition, a "distal side" used in the following description is a side on which the fingertip of the right hand H1 is disposed (left side of Figs. 1 and 5E) in a state in which hemostatic device 100 is attached to the right hand H1 of the patient.

### <Covering member>

As illustrated in Figs. 1 and 2, the covering member 110 includes the main body 120 to which the inflatable member 160 is connected, a first arm portion 130 protruding from the main body 120, and a second arm portion 140 protruding from the main body 120 while forming an obtuse angle with a longitudinal direction of the first arm portion 130.

The first arm portion 130 has an inclined portion 131 located on an end portion side connected to a first region 121a of the main body 120, and a convex portion 133a protruding in a width direction of the first arm portion 130 at an end portion 133 opposite to a side where the inclined portion 131 is disposed.

As illustrated in Figs. 1 and 2, the longitudinal direction of the first arm portion 130 is a vertical direction of Figs. 1 and 2 in which the first arm portion 130 extends in a state in which the first arm portion 130 is extended without attaching the hemostatic device 100 to the right hand H1 of the patient. In addition, a width direction of the first arm portion 130 is a direction intersecting the longitudinal direction of the first arm portion 130 on the plan views illustrated in Figs. 1 and 2 and a left-right direction of Figs. 1 and 2. Similarly, a longitudinal direction of the second arm portion 140 is the vertical direction of Figs. 1 and 2 in which the second arm portion 140 extends in a state in which the second arm portion 140 is extended without attaching the hemostatic device 100 to the right hand H1 of the patient, and a width direction of the second arm portion 140 is the left-right direction of Figs. 1 and 2 intersecting the longitudinal direction of the second arm portion 140 on the plan views illustrated in Figs. 1 and 2.

The inclined portion 131 and the convex portion 133a of the first arm portion 130 are formed of a flexible band-shaped member that can wrap the first arm portion 130 around the right hand H1 of the patient.

As illustrated in Fig. 1, the inclined portion 131 is inclined in a direction away from a distal side of the main body 120. A convex portion 113a protrudes toward the distal side of the main body 120 in a direction intersecting the longitudinal direction of the first arm portion 130. Further, the convex portion 113a is inclined in a direction toward the main body 120 side. For example, a width W3 of the convex portion 133a can be set to 25 mm to 40 mm.

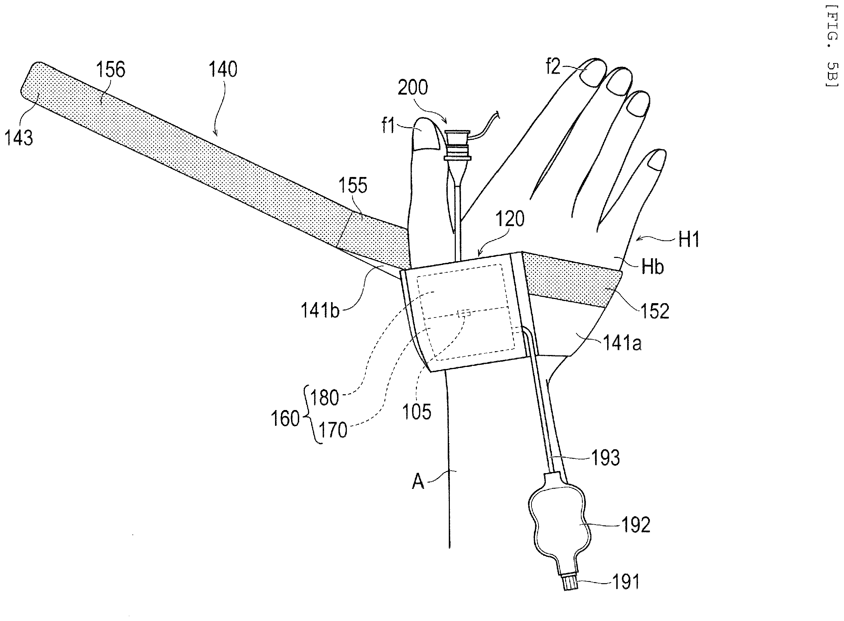

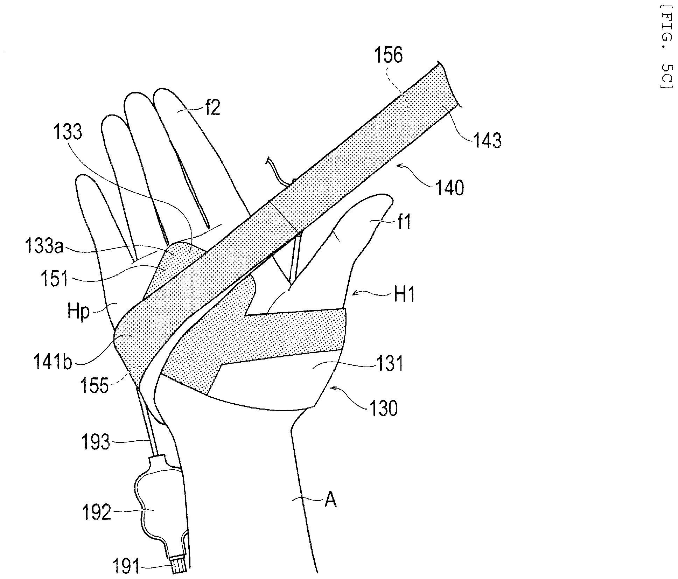

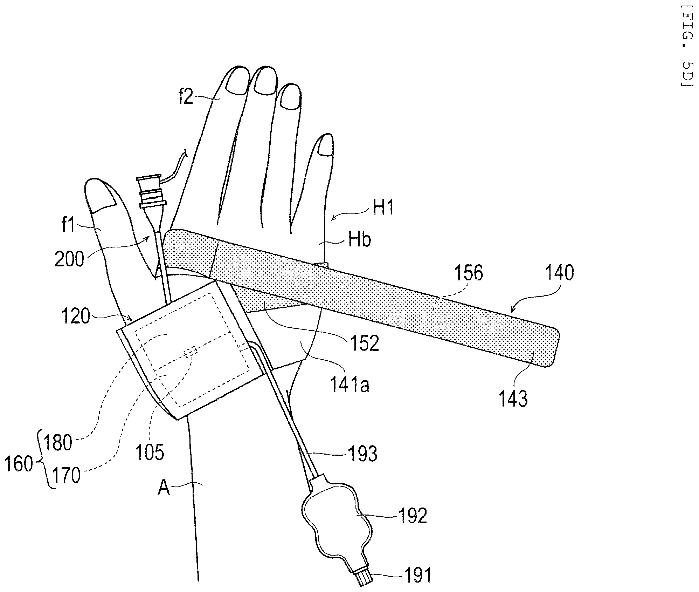

As illustrated in Figs. 1 and 2, the second arm portion 140 is formed longer than the first arm portion 130. Further, as illustrated in Figs. 5B, 5C, and 5D, the second arm portion 140 is configured to be secured to the convex portion 133a of the first arm portion 130 and secured to the second arm portion 140 by passing between fingers f1 and f2 of the patient in a state in which the second arm portion 140 is wrapped around a limb of the patient.

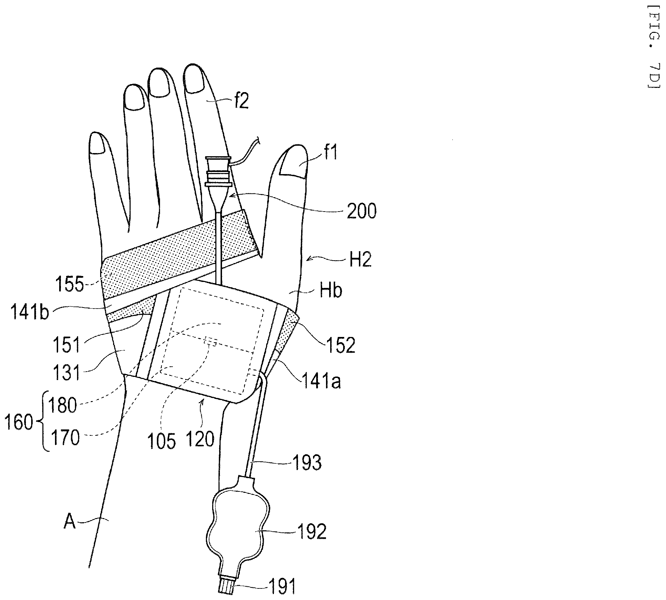

The second arm portion 140 is formed of a flexible band-shaped member that can be wrapped around the limb of the patient. Note that the limb around which the second arm portion 140 is wrapped includes, for example, a part of the right hand H1 of the patient, a part of the forearm A, and a part of a wrist. Further, a space between the fingers (inter-finger portion) through which the second arm portion 140 passes is, for example, a space between the thumb f1 and the index finger f2 of the right hand H1 of the patient. However, a position of the limb around which the second arm portion 140 is wrapped or a position of the inter-finger portion through which the second arm portion 140 is passed is not particularly limited. Note that the hemostatic device 100 according to the present embodiment can be attached to the left hand H2 in the same manner as each part of the right hand H1 described above (see Fig. 7D).

As illustrated in Fig. 1, a magnitude relationship between a length L1 of the first arm portion 130 and a length L2 of the second arm portion 140 can be defined by lineal distances L1 and L2 of the respective arm portions 130 and 140 in a state in which the respective arm portions 130 and 140 are extended without attaching the hemostatic device 100 to the right hand H1 of the patient. Note that, for example, the length L1 of the first arm portion 130 can be set to 80 mm to 400 mm. In addition, for example, the length L2 of the second arm portion 140 can be set to 350 mm to 400 mm.

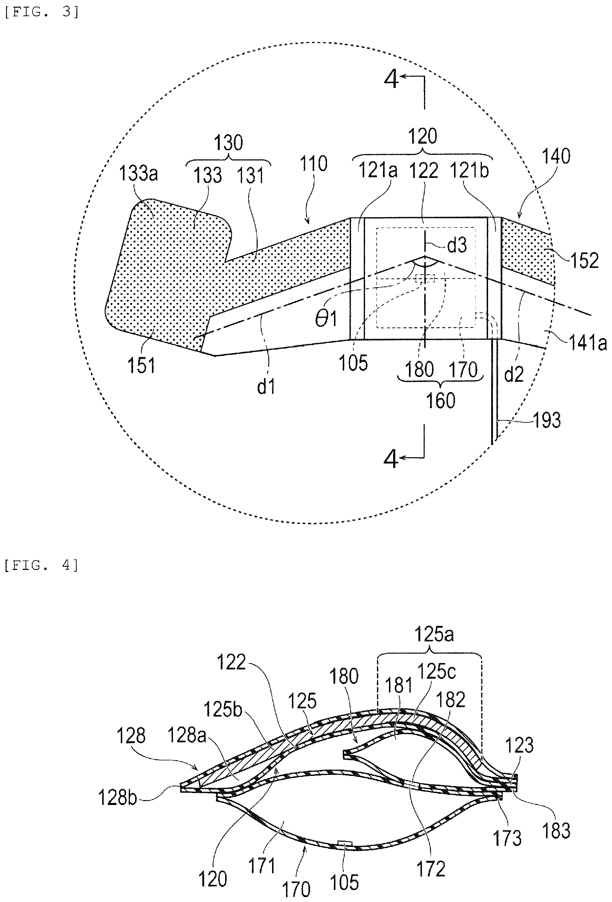

In the present embodiment, as illustrated in Fig. 3, an obtuse angle θ1 formed between the longitudinal direction of the first arm portion 130 and the longitudinal direction of the second arm portion 140 can be defined as an angle formed by intersection of a straight line d1 extending substantially parallel to a longitudinal direction of the inclined portion 131 of the first arm portion 130 and a straight line d2 extending substantially parallel to a longitudinal direction of a first inclined portion 141a of the second arm portion 140 on the main body 120. For example, the obtuse angle θ1 can be set to 120° to 170°.

As described above, in the hemostatic device 100, the first arm portion 130 and the second arm portion 140 form an obtuse angle on the plan views illustrated in Figs. 1 and 3. Therefore, the first arm portion 130 is inclined and extends in a direction away from the distal side of the main body 120. Similarly, the second arm portion 140 is inclined and extends in a direction away from the distal side of the main body 120. Therefore, the first arm portion 130 and the second arm portion 140 extend from the distal side of the main body 120 toward the proximal side so as to spread in the shape of the kanji character for the number eight without intersecting each other.

Note that an angle formed by a straight line d3 passing through a center position of the main body 120 (corresponding to a center line of a first inflatable portion 170) and a straight line d1 along the longitudinal direction of the first arm portion 130 may be the same as or different from an angle formed by the straight line d3 passing through the center portion of the main body 120 and a straight line d2 along the longitudinal direction of the second arm portion 140.

As illustrated in Figs. 1 and 2, the second arm portion 140 has an inclined portion 141 and a second arm end portion 143 that is continuously formed with the inclined portion 141 and forms an end portion of the second arm portion 140.

The inclined portion 141 of the second arm portion 140 has the first inclined portion 141a connected to the first region 121a of the main body 120 and a second inclined portion 141b extending between the first inclined portion 141a and the second arm end portion 143.

The first inclined portion 141a is inclined in a direction away from the distal side of the main body 120. Similarly to the first inclined portion 141a, the second inclined portion 141b is inclined in a direction away from the distal side of the main body 120.

A width of the first inclined portion 141a gradually decreases from the first region 121b side of the main body 120 toward the second inclined portion 141b side. A width of the second inclined portion 141b gradually decreases from the first inclined portion 141a side to the second arm end portion 143 side.

The second arm end portion 143 extends linearly along an extending direction of the second arm portion 140 with a substantially constant width. Note that the second arm end portion 143 is a portion including a predetermined range in the extending direction of the second arm portion 140 from an end portion (terminal) of the second arm portion 140.

As illustrated in Fig. 1, a width W1 of the inclined portion 141 is larger than a width W2 of the second arm end portion 143. The width W1 is a width of the inclined portion (the first inclined portion 141a and the second inclined portion 141b) 140 at an arbitrary position.

For example, the width W1 of the inclined portion 141 can be set to 15 mm to 45 mm. Further, for example, the width W2 of the second arm end portion 143 can be set to 10 mm to 20 mm. A maximum value of the width W1 of the inclined portion 141 illustrated above is a maximum value of the first inclined portion 141a, and a minimum value of the width W1 of the inclined portion 141 is a minimum value of the second inclined portion 141b. Note that the inclined portion 141 can be formed by one inclined portion extending from the first region 121b side of the main body 120 to the second arm end portion 143. Even in such a configuration, the width W1 of the inclined portion 141 illustrated above can be adopted.

In the first arm portion 130 and the second arm portion 140, for example, it is possible to provide an entry portion that allows the operator, etc. to enter the amount of air injected into the inflatable member 160, a hemostatic time, etc. in a procedure using the hemostatic device 100. For example, the entry portion can be made of a material on which a character, etc. can be written using ink, etc. provided in a known pen.

As illustrated in Fig. 3, the main body 120 of the covering member 110 has the first regions 121a and 121b, and a second region 122 in which the inflatable member 160 is disposed unlike the first regions 121a and 121b.

The first region 121a is disposed between the second region 122 and the first arm portion 130. The first region 121b is disposed between the second region 122 and the second arm portion 140.

As illustrated in Fig. 4, the second region 122 of the main body 120 has the support member 125 having a larger rigidity than that of the first regions 121a and 121b of the main body 120. In the support member 125, both side portion sides located in the left-right direction are adjacent to the first regions 121a and 121b on the plan view illustrated in Fig. 3.

As illustrated in Fig. 4, the support member 125 is inserted into an insertion portion 128a disposed on an outer surface side of the second region 122 (surface opposite to a side where each of the inflatable portions 170 and 180 is disposed in the second region 122, which is a surface on an upper side of Fig. 4).

The insertion portion 128a is a space formed between the cover member 128 disposed to cover a part of the outer surface of the main body 120 and the main body 120.

An insertion port 128b communicating with the insertion portion 128a is formed on the proximal side (lower side of Fig. 3 and left side of Fig. 4) of the main body 120. The support member 125 can be inserted into the insertion portion 128a via the insertion port 128b.

For example, the cover member 128 can be connected to the covering member 110 by adhesion or welding. In the present embodiment, the cover member 128 is connected to the covering member 110 at three sides other than a part where the insertion port 128b is formed.

As illustrated in Fig. 4, the support member 125 has a curved portion 125a formed on the distal side of the main body 120. The curved portion 125a has a cross-sectional shape protruding upward so as to be separated from the main body 120 on the cross-sectional view illustrated in Fig. 4. Note that the distal side of the man body 120 means an upper side of the center position with reference to a center position (position where a marker portion 105 is disposed in the present embodiment) on the plan view of the inflatable member 160 illustrated in Fig. 3.

When the inflatable member 160 is inflated, the curved portion 125a of the support member 125 wraps the vicinity of the end portion of the inflatable member 160 on the distal side (an end portion 173 of the first inflatable portion 170 and an end portion 183 of the second inflatable portion 180), so that the compressive force of the inflatable member 160 is directed toward the central side of the support member 125 (center side in the left-right direction of Fig. 4). Thus, the compressive force of the inflatable member 160 is inhibited from escaping to the outside of the support member 125. As a result, the hemostatic device 100 can suppress a decrease in the compressive force of the inflatable member 160 on the puncture site t1, and thus it is possible to appropriately maintain the compressive force of the inflatable member 160 on the puncture site t1.

Note that, for example, an apex 125c of the curved portion 125a of the support member 125 (a part of the curved portion 125a most distant from the inflatable member 160) can be disposed on the distal side of the main body 120 with respect to the center position of the inflatable member 160. By disposing the apex 125c of the curved portion 125a at the position described above, the support member 125 can reliably wrap the vicinity of the end portion of the inflatable member 160 on the distal side when the inflatable member 160 inflates, and thus it is possible to effectively inhibit the compressive force of the inflatable member 160 from escaping to the outside of the support member 125.

As illustrated in Fig. 4, the support member 125 has an inclined portion 125b extending from the curved portion 125a toward the proximal side of the main body 120. The inclined portion 125b extends substantially linearly toward the proximal side of the main body 120.

A width direction dimension (vertical direction dimension of Fig. 3) of the support member 125 is not particularly limited. However, for example, it is preferable to adopt a size that allows the proximal end of the support member 125 to be disposed near the proximal end of the main body 120 in a state in which a distal end of the support member 125 is disposed near a distal end of the auxiliary member 180 as illustrated in Fig. 4. Further, a longitudinal direction dimension (left-right direction dimension of Fig. 3) of the support member 125 is not particularly limited. However, for example, when the first regions 121a and 121b are formed on the main body 120, it is preferable to adopt a dimension that allows formation of the first regions 121a and 121b having desired sizes on both side portion sides of the main body 120.

Note that, for example, the support member 125 may be disposed on the inner surface side of the main body 120 of the covering member 110 (surface on a side where each of the inflatable portions 170 and 180 is disposed, which is a surface on a lower side of Fig. 4). When the support member 125 is disposed in this way, connection of the cover member 128 to the main body 120 can be omitted, and each of the inflatable portions 170 and 180 can be fixed to the support member 125.

In the present embodiment, the first regions 121a and 121b and the second region 122 of the main body 120 are integrally formed of one member. However, the first region 121a and 121b and the second region 122 may be configured by connecting different members.

In the second region 122 of the main body 120, the cover member 128, the support member 125, the first inflatable portion 170, and the second inflatable portion 180, a portion overlapping the marker portion 105 in the plan view illustrated in Figs. 1 and 3 and surroundings thereof are preferably transparent (including colored transparent, colorless transparent, and translucent). By adopting such a configuration, the operator can visually recognize the puncture site t1 from the outer surface side of the main body 120 even when the marker portion 105 is superposed on the puncture site t1.

Note that it is preferable that the first regions 121a and 121b of the main body 120 are made of a material having higher elasticity than that of the first arm portion 130 and the second arm portion 140. In this way, when the hemostatic device 100 is attached to the right hand H1 of the patient, by wrapping each of the arm portions 130 and 140 around the limb of the patient, the first region 121a is pulled toward the first arm portion 130 side and extended, and the first region 121b is pulled toward the second arm portion 140 side and extended. As a result, a physical property of the main body 120 changes at each boundary between the first arm portion 130 and the second arm portion 140 to allow the main body to be deformed easily, and thus the first arm portion 130 and the second arm portion 140 can be easily disposed on the right hand H1 of the patient in a state where the main body 120 is disposed on the puncture site t1 formed on the right hand H1 of the patient.

Further, a material of the support member 125 is preferably more rigid than a material of each of the first arm portion 130 and the second arm portion 140. As a result, when the inflatable member 160 inflates, the support member 125 can suppress rising of the main body 120 by the inflatable member 160 due to the rigidity of the support member 125. Further, since the first arm portion 130 and the second arm portion 140 are configured to be more flexible than the support member 125, the hemostatic device 100 can be easily attached to the patient along the limb of the patient when being attached to the patient.

Note that in the present embodiment, in the covering member 110, each of the main body 120, the first arm portion 130, and the second arm portion 140 is formed as a separate member. When each portion of the covering member 110 is configured as a separate member in this way, each of the main body 120, the first arm portion 130, and the second arm portion 140 can be connected by, for example, adhesion, welding, etc. However, in the covering member 110, an arbitrary part of the main body 120, the first arm portion 130, and the second arm portion 140 may be integrally formed of one member.

The material used for the main body 120 of the covering member 110 is not particularly limited. Examples thereof include polyvinyl chloride, polyolefin such as polyethylene, polypropylene, polybutadiene, or ethylene-vinyl acetate copolymer (EVA), polyester such as polyethylene terephthalate (PET) or polybutylene terephthalate (PBT), polyvinylidene chloride, silicone, polyurethane, various thermoplastic elastomers such polyamide elastomer, polyurethane elastomer, and polyester elastomer, nylon, nylon elastomer, or any combination thereof (blended resin, polymer alloy, laminate, etc.).

The material used for the cover member 128 is not particularly limited, and examples thereof include the same materials as those exemplified as the material of the covering member 110.

The material used for the support member 125 preferably has a higher rigidity than that of the material used for the first regions 121a and 121b of the main body 120 of the covering member 110. Examples of such a material may include acrylic resin, polyvinyl chloride (especially hard polyvinyl chloride), polyolefin such as polyethylene, polypropylene, or polybutadiene, polystyrene, poly-(4-methylpentene-1), polycarbonate, ABS resin, polymethylmethacrylate (PMMA), polyacetal, polyacrylate, polyacrylonitrile, polyvinylidene fluoride, ionomer, acrylonitrile-butadiene-styrene copolymer, polyester such as polyethylene terephthalate (PET) or polybutylene terephthalate (PBT), butadiene-styrene copolymer, aromatic or aliphatic polyamide, fluorine-based resin such as polytetrafluoroethylene, etc.

The material used for the first arm portion 130 and the second arm portion 140 of the covering member 110 is not particularly limited. Examples thereof may include the same materials as the materials exemplified as the main body 120 of the covering member 110, woven fabric, nonwoven fabric, felt, cloth, knitted fabric, and paper.

### <Securing member>

As illustrated in Figs. 1 and 2, the hemostatic device 100 includes six securing members of a first securing member 151, a second securing member 152, a third securing member 153, a fourth securing member 154, a fifth securing member 155, and a sixth securing member 156.

As illustrated in Fig. 1, the first securing member 151 is disposed on the outer surface of the first arm portion 130. The first securing member 151 is disposed on a part of the inclined portion 131 on the distal side and the entire end portion 133.

As illustrated in Fig. 1, the second securing member 152 and the third securing member 153 are disposed on the outer surface of the second arm portion 140. The second securing member 152 is disposed on a part of the first inclined portion 141a on the distal side and a part of the second inclined portion 141b on the distal side. The third securing member 153 is disposed on the entire second arm end portion 143.

As illustrated in Fig. 2, the fourth securing member 154 is disposed on the inner surface of the first arm portion 130. The fourth securing member 154 is disposed on a part of the inclined portion 131 on the distal side and the entire end portion 133.

As illustrated in Fig. 2, the fifth securing member 155 and the sixth securing member 156 are disposed on the outer surface of the second arm portion 140. The fifth securing member 155 is disposed on a part of the first inclined portion 141a on the distal side and a part of the second inclined portion 141b on the distal side. The sixth securing member 156 is disposed on the entire second arm end portion 143.

The first securing member 151 and the second securing member 152 are formed on a male side of a surface fastener. The third securing member 153, the fourth securing member 154, the fifth securing member 155, and the sixth securing member 156 are formed on a female side of the surface fastener. The surface fastener is a fastener that is removable in terms of surface, and is, for example, Magic Tape (registered trademark) or Velcro (registered trademark).

Note that a specific configuration of each of the securing members 151, 152, 153, 154, 155, and 156 is not limited as long as the second arm portion 140 wrapped around the limb of the patient can be secured to the convex portion 133a, and a part of the second arm portion 140 passed between the thumb f1 and the index finger f2 can be secured to the second arm portion 140. For example, some of the securing members 151, 152, 153, 154, 155, and 156 can be omitted, and positions where the securing members are disposed on the respective arm portions 130 and 140 can be changed as appropriate. Further, when each of the securing members 151, 152, 153, 154, 155, and 156 includes a surface fastener, a male side and a female side of the surface fastener may be interchanged. Further, for example, each of the securing members 151, 152, 153, 154, 155, and 156 may be a snap, a button, a clip, a frame member in which a hole is formed, etc.

### <Inflatable member>

As illustrated in Figs. 3 and 4, the inflatable member 160 has the first inflatable portion 170 and the deformable auxiliary member 180 having a smaller outer shape (outer shape on the plan view of Fig. 3) than that of the first inflatable portion 170.

The auxiliary member 180 is disposed to overlap the first inflatable portion 170 on the distal side of the main body 120.

The first inflatable portion 170 has a lumen 171 into which a fluid can be injected, and a communication hole 172 formed at a position facing the auxiliary member 180.

In the present embodiment, the auxiliary member 180 is a second inflatable portion configured to be inflated by injection of a fluid. Hereinafter, the auxiliary member 180 will be referred to as a second inflatable portion.

The second inflatable portion 180 has a lumen 181 into which a fluid can be injected, and a communication hole 182 disposed at a position facing the communication hole 172 of the first inflatable portion 170.

The lumen 181 of the second inflatable portion 180 communicates with the lumen 171 of the first inflatable portion 170 through the communication hole 182 of the second inflatable portion 180 and the communication hole 172 of the first inflatable portion 170.

As illustrated in Fig. 3, the second inflatable portion 180 is disposed to have bilateral symmetry with respect to the center line d3 of the first inflatable portion 170 (inflatable member 160). Further, the first securing member 151, the second securing member 152, and the third securing member 153 described above are disposed on the outer surface of the first arm portion 130 and the outer surface of the second arm portion 140 opposing to each other with the main body 120 interposed therebetween.

In the present embodiment, the second inflatable portion 180 has a substantially square shape. Therefore, in the second inflatable portion 180, a center position of the square in the left-right direction is disposed at the center position of the main body 120. Further, the marker portion 105, which will be described later, is disposed at the center position of the second inflatable portion 180.

The second inflatable portion 180 is fixed to the inner surface of the covering member 110 (the inner surface of the main body 120 of the covering member 110). Specifically, the end portion 183 located on the distal side of the second inflatable portion 180 is fixed to the inner surface of the covering member 110.

In the first inflatable portion 170, the periphery of the communication hole 172 of the first inflatable portion 170 is fixed to the periphery of the communication hole 182 of the second inflatable portion 180. Specifically, in the first inflatable portion 170, only a certain range around each of the communication holes 172 and 182 located near the central portion of the second inflatable portion 180 is fixed to the second inflatable portion 180. As described above, the first inflatable portion 170 is not directly connected to the covering member 110, and is indirectly connected to the covering member 110 via the second inflatable portion 180. As a result, in the second inflatable portion 180, the peripheral edge of the second inflatable portion 180 is not fixed to the first inflatable portion 170, and thus the outer shape of the second inflatable portion 180 is freely deformed. For this reason, even when the patient bends the finger (for example, the thumb f1) or the wrist upward (to the dorsal side Hb of the hand), the hemostatic device 100 can maintain a large area in which the second inflatable portion 180 compresses the first inflatable portion 170. Therefore, the hemostatic device 100 can effectively apply a compressive force to the puncture site t1 from the inflatable member 160. Note that in the first inflatable portion 170 and the second inflatable portion 180, for example, the first inflatable portion 170 and the second inflatable portion 180 may be integrally connected to the covering member 110 by fixing the end portion 183 of the second inflatable portion 180 to the covering member 110 in a state where the first inflatable portion 170 and the second inflatable portion 180 are connected.

The inflatable member 160 may be fixed to the inner surface of the covering member 110 at a position different from that in Fig. 3 as long as the inflatable member 160 is fixed to the covering member 110 on the curved portion 125a side of the support member 125. Specifically, a part or the whole of the outer surface side of the second inflatable portion 180 may be fixed to the inner surface of the covering member 110 on the curved portion 125a side of the support member 125. Even in such a configuration, in the hemostatic device 100, a compression direction of the inflatable member 160 is directed to the center side of the first inflatable portion 170 (the center side in the left-right direction of Fig. 4) by the curved portion 125a of the support member 125. Therefore, the hemostatic device 100 suppresses a decrease in the compressive force due to the inflatable member 160 even when a gap is generated between the main body 120 and the body surface of the hand H due to movement of the finger of the patient or bending of the wrist.

Further, it is preferable that the second inflatable portion 180 is located inside the curved portion 125a of the support member 125 and is disposed to overlap the first inflatable portion 170. As a result, since the second inflatable portion 180 is located inside the curved portion 125a of the support member 125, the compressive force of the second inflatable portion 180 is directed toward the center side of the support member 125 by the curved portion 125a, and inhibited from escaping to the outside of the support member 125. Further, when the second inflatable portion 180 is disposed to overlap the first inflatable portion 170 on the curved portion 125a side of the support member 125, the second inflatable portion 180 can prevent the first inflatable portion 170 from rising on the distal side of the right hand H1 or the left hand H2 while preventing position shift of the first inflatable portion 170. Therefore, the hemostatic device 100 can more reliably prevent the inflatable member 160 from rising, and can suppress a decrease in the compressive force applied to the puncture site t1 by the inflatable member 160.

The first inflatable portion 170 has a substantially square shape on the plan view illustrated in Fig. 2. The second inflatable portion 180 has a substantially rectangular shape that includes a set of long sides having substantially the same length as that of one side of the first inflatable portion 170 and a set of short sides having a length which is approximately half a length of one side of the first inflatable portion 170.

The lumen 171 of the first inflatable portion 170 communicates with a lumen of a tube 193 for supplying a fluid such as air to the first inflatable portion 170. As illustrated in Figs. 1 and 3, the tube 193 is connected to the first inflatable portion 170 on the proximal side of the first inflatable portion 170. The tube 193 is pulled out to the outside of the main body 120 through the inner surface side of the main body 120 of the covering member 110. A position where the tube 193 is pulled out from the first inflatable portion 170 is not particularly limited. However, as illustrated in Fig. 3, when the tube 193 is pulled out to the proximal side of the main body 120, so that the hemostatic device 100 is attached to the patient, the tube 193 is disposed laterally to the right hand H1 (in a direction intersecting a direction in which the finger of the right hand H1 extends) (see Fig. 5B). For this reason, when the hemostatic device 100 is attached to the patient, the tube 193 can be prevented from interfering with the introducer 200.

Note that the tube 193 may be connected to the second inflatable portion 180. Further, a position at which the tube 193 is pulled to the outside of the main body 120 can be appropriately changed.

As illustrated in Figs. 3 and 4, the hemostatic device 100 has the marker portion 105 for aligning the inflatable member 160 with respect to the puncture site t1.

The marker portion 105 is disposed at a position corresponding to a substantially center position (center position on the plan view illustrated in Fig. 2) of the first inflatable portion 170.

As illustrated in Fig. 4, for example, the marker portion 105 can be disposed on an inner surface of a side surface (inner surface) of the first inflatable portion 170 disposed to face the body surface. However, for example, the marker portion 105 may be disposed on an internal surface of a surface (outer surface) opposite to the side surface of the first inflatable portion 170 disposed to face the body surface or an external surface thereof, an internal surface or an external surface of the main body 120 of the covering member 110, an internal surface or an external surface of the support member 125, an internal surface or an external surface of the cover member 128, etc. In addition, when a center portion of the first inflatable portion 170 and an end portion of the second inflatable portion 180 (an end portion on the proximal side, which is an end portion on the left side of Fig. 4) are disposed to overlap each other on the cross-sectional view illustrated in Fig. 4, the marker portion 105 may be disposed on an external surface of the end portion of the second inflatable portion 180.

For example, the marker portion 105 preferably includes a transparent central portion and a colored linear frame portion surrounding the central portion. In this way, the operator can dispose the marker portion 105 at the puncture site t1 while confirming the puncture site t1 through the transparent central portion of the marker portion 105. For this reason, the operator can easily dispose the center position of the first inflatable portion 170 at the puncture site t1 using the marker portion 105. Note that, for example, the marker portion 105 may be formed only by the colored central portion without having the frame portion. Further, a specific shape and color of the marker portion 105, a formation method on each portion of the hemostatic device 100, etc. are not particularly limited.

In the present embodiment, the first inflatable portion 170 is formed of two sheet-shaped members. For example, the first inflatable portion 170 can be formed by forming the lumen 171 between two sheet-shaped members formed in a substantially rectangular shape and bonding outer peripheral edges of the two sheet-shaped members in this state. Similarly to the first inflatable portion 170, the second inflatable portion 180 can be formed of two substantially rectangular sheet-shaped members bonded together.

A method of bonding the sheet-shaped members forming the first inflatable portion 170, and a method of bonding the sheet-shaped members forming the second inflatable portion 180 are not particularly limited. For example, it is possible to adopt adhesion or welding. Further, a method of connecting the second inflatable portion 180 and the main body 120 of the covering member 110 is not particularly limited. For example, adhesion or welding can be adopted. Further, a method of fixing the first inflatable portion 170 and the second inflatable portion 180 is not particularly limited. For example, adhesion or welding can be adopted.

Note that the first inflatable portion 170 and the second inflatable portion 180 may not have a structure in which a plurality of sheet-shaped members is bonded. The first inflatable portion 170 and the second inflatable portion 180 may be formed of, for example, one bag-shaped member in which a space into which a fluid can flow is formed.

A material used for the first inflatable portion 170 and the second inflatable portion 180 is not particularly limited, and examples thereof may include the same materials as those exemplified as the material of the covering member 110.

### <Injection portion>

As illustrated in Fig. 1, the hemostatic device 100 has an injection portion 191 for injecting a fluid into the inflatable member 160 (the first inflatable portion 170 and the second inflatable portion 180).

The injection portion 191 includes a connector having an incorporated check valve (not illustrated). A syringe (not illustrated) can be connected to the injection portion 191.

A cushioning member 192 having an inflatable space is disposed between the injection portion 191 and the inflatable member 160. The cushioning member 192 includes a flexible bag-shaped member having a space formed inside. Note that the cushioning member 192 may be provided with an arrow-shaped marker indicating a direction in which the syringe is inserted into the injection portion 191.

The injection portion 191 is connected to one end side of the cushioning member 192. A lumen of the injection portion 191 communicates with a space in the cushioning member 192. However, while the check valve incorporated in the injection portion 191 is closed, communication between the lumen of the injection portion 191 and the space in the cushioning member 192 is cut off.

A flexible tube 193 is connected to the other end side of the cushioning member 192. A lumen of the tube 193 communicates with the space in the cushioning member 192. Further, in the tube 193, the other end portion opposite to one end portion connected to the cushioning member 192 is connected to the first inflatable portion 170. The lumen of the tube 193 communicates with the lumen 171 of the first inflatable portion 170.

For example, the other end portion of the tube 193 can be connected to the first inflatable portion 170 using an adhesive, etc. while being interposed between the two sheet-shaped members forming the first inflatable portion 170. Note that, in the sheet-shaped members forming the first inflatable portion 170, for example, convex portions partially protruding outward from the sheet-shaped members may be formed at parts interposing the tube 193 therebetween.

To inflate the first inflatable portion 170 and the second inflatable portion 180, the operator inserts a distal tubular portion of a syringe (not illustrated) into the injection portion 191 to open the check valve. The operator injects air in the syringe into the lumen 171 of the first inflatable portion 170 by pushing a plunger of the syringe with the check valve of the injection portion 191 open.

When air is injected into the lumen 171 of the first inflatable portion 170, the first inflatable portion 170 inflates. Further, the air injected into the lumen 171 of the first inflatable portion 170 flows into the lumen 181 of the second inflatable portion 180 via the communication hole 172 of the first inflatable portion 170 and the communication hole 182 of the second inflatable portion 180. When air flows into the lumen 181 of the second inflatable portion 180, the second inflatable portion 180 inflates. When the first inflatable portion 170 and the second inflatable portion 180 inflate, the cushioning member 192 communicating with the lumen 171 of the first inflatable portion 170 via the tube 193 inflates.

The space in cushioning member 192 and the lumen 171 of the first inflatable portion 170 are in communication with each other via the tube 193 at all times. The injection portion 191 maintains the check valve incorporated in the injection portion 191 in a closed state when the syringe is not inserted into the injection portion 191 to prevent air from leaking from the injection portion 191. For this reason, when the internal pressure of the inflatable member 160 increases due to movement of the right hand H of the patient, etc., the air in the lumen 171 of the first inflatable portion 170 and the lumen 181 of the second inflatable portion 180 moves to the cushioning member 192 side which is not pressed against the puncture site t1 by the covering member 110. In this way, the compressive force applied to the puncture site t1 by the inflatable member 160 is adjusted, and thus the compressive force to the puncture site t1 by the inflatable member 160 can be appropriately maintained.

When the operator contracts the first inflatable portion 170 and the second inflatable portion 180, the operator inserts the distal tubular portion of the syringe into the injection portion 191 and pulls the plunger of the syringe. By performing the above operation, the operator can discharge the air in the first inflatable portion 170 and the air in the second inflatable portion 180 to the syringe.

Note that when the operator, etc. inflates the inflatable member 160, the operator, etc. can visually confirm that the first inflatable portion 170 and the second inflatable portion 180 can be pressurized without leakage of air by confirming expansion of the cushioning member 192.

Next, a usage example of the hemostatic device 100 will be described with reference to Figs. 5A to 5E. In the following, a description will be given of an example of a procedure for attaching the hemostatic device 100 to the right hand H1 of the patient on which the puncture site t1 is formed.

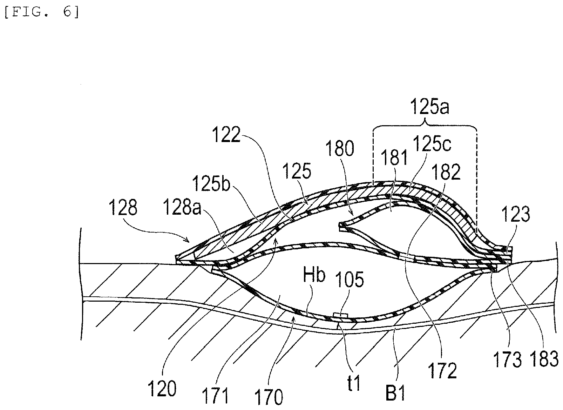

Fig. 5A illustrates a state in which various procedures are performed by inserting the sheath tube of the introducer 200 into the distal radial artery side of the palmar artery B1 via the puncture site t1 (see Fig. 6) formed on the dorsal side Hb of the right hand H1 of the patient. Further, Fig. 5A illustrates a state in which a part of the sheath tube of the introducer 200 is pulled out from the puncture site t1 after the above procedure is completed.

At the start of hemostasis, as illustrated in Fig. 5A, the operator, etc. disposes the main body 120 of the covering member 110 so that the main body 120 overlaps a side of the dorsal side Hb of the right hand H1. In this instance, the marker portion 105 disposed at a substantially center position of the first inflatable portion 170 is disposed on the puncture site t1.

Subsequently, as illustrated in Figs. 5B and 5C, the operator, etc. wraps the inclined portion (the first inclined portion 141a and the second inclined portion 141b) 141 of the second arm portion 140 along the right hand H1 of the patient while wrapping the convex portion 133a of the first arm portion 130 around the right hand H1 of the patient. In this instance, the operator, etc. secures the convex portion 133a and the second arm portion 140 via the respective securing members 151 and 155 by bringing the fifth securing member 155 (female side of the surface fastener) disposed on the inner surface of the inclined portion 141 into contact with the first securing member 151 (male side of the surface fastener) disposed on the outer surface of the convex portion 133a of the first arm portion 130 on the palm Hp side of the right hand H1 of the patient.

When the second arm portion 140 is secured to the convex portion 133a, the operator, etc. can confirm the position of the convex portion 133a and the shape of the convex portion 133a by the feel of the fingers. Therefore, the operator, etc. can guide the second arm portion 140 to the convex portion 133a disposed on the palm Hp side of the right hand H1 of the patient and recognize a direction in which the second arm portion 140 is disposed on the convex portion 133a, and thus the second arm portion 140 can be easily secured to the convex portion 133a.

Subsequently, as illustrated in Fig. 5D, the operator, etc. passes the second arm portion 140 between the thumb f1 and the index finger f2 of the right hand H1 of the patient to dispose the second arm end portion 143 on the side of the dorsal side Hb of the hand H of the patient. Subsequently, operator, etc. secures the first inclined portion 141a and the second arm end portion 143 via the respective securing members 152 and 156 by bringing the sixth securing member 156 (female side of the surface fastener) disposed on the inner surface of the second arm end portion 143 into contact with the second securing member 152 (male side of the surface fastener) disposed on the outer surface of the first inclined portion 141a on the side of the dorsal side Hb of the right hand H1 of the patient.

Subsequently, the operator, etc. wraps a portion (surplus portion) of the second arm end portion 143 not secured to the first inclined portion 141a along a circumferential direction of the right hand H1 of the patient, thereby further securing the second arm end portion 143 to the convex portion 133a. In this way, the hemostatic device 100 can prevent the second arm end portion 143 from being caught in a surrounding article, etc. while the hemostatic device 100 is attached to the operator, etc.

The operator, etc. can secure the hemostatic device 100 to the right hand H1 of the patient by the above procedure. Fig. 5E illustrates a state in which the hemostatic device 100 is attached to the right hand H1 of the patient. Note that Fig. 5E illustrates a state in which the introducer 200 is removed from the puncture site t1.

Next, the operator, etc. connects the syringe to the injection portion 191 and injects air into the first inflatable portion 170 to inflate the first inflatable portion 170 and the second inflatable portion 180. In the hemostatic device 100, when the first inflatable portion 170 and the second inflatable portion 180 are inflated, the first inflatable portion 170 applies a compressive force to the puncture site t1. After inflating the respective inflatable portions 170 and 180, as illustrated in Fig. 5E, the operator, etc. removes the sheath tube of the introducer 200 from the puncture site t1. In this instance, as illustrated in Fig. 5D, since the second arm portion 140 is disposed between the thumb f1 and the index finger f2 of the right hand H1 of the patient, the hemostatic device 100 does not have an arm portion protruding from the main body 120 in a region where the curved portion 125a of the support member 125 and the second inflatable portion 180 are located in the main body 120. For this reason, after inflating the respective inflatable portions 170 and 180, as illustrated in Fig. 5E, the operator, etc. can remove the sheath tube of the introducer 200 from the puncture site t1.

The operator, etc. confirms that there is no bleeding from the puncture site t1 during hemostasis using the hemostatic device 100. When there is bleeding from the puncture site t1, the operator, etc. adjusts the amount of air injected into each of the inflatable portions 170 and 180.

After a certain period of time from the start of hemostasis, the operator, etc. gradually depressurizes each of the inflatable portions 170 and 180 to confirm that hemostasis is properly performed on the puncture site t1. After the hemostasis on the puncture site t1 is completed, the operator, etc. sufficiently depressurizes each of the inflatable portions 170 and 180. Then, the operator, etc. releases securing of the hemostatic device 100 by the first arm portion 130 and the second arm portion 140, and removes the hemostatic device 100 from the hand H of the patient.

As illustrated in Fig. 5E, during hemostasis, the hemostatic device 100 is firmly secured to the right hand H1 of the patient by the second arm portion 140 passed between the thumb f1 and the index finger f2 of the right hand H1 of the patient while being wrapped around the limb of the patient. For this reason, since each finger is not covered by the covering member 110 when the hemostatic device 100 is attached to the right hand H1, the patient can freely move the finger while hemostasis is performed.

By proceeding with an attaching operation according to a procedure described with reference to Figs. 5A to 5E, the operator, etc. can use the first arm portion 130 and the second arm portion 140 to easily attach the hemostatic device 100 to the patient in a short time. Further, the hemostatic device 100 can secure the covering member 110 to the right hand H1 of the patient using the securing members 151, 152, 153, 154, 155, and 156 disposed on the respective arm portions 130 and 140. Therefore, the hemostatic device 100 can reduce a burden on the skin of the patient during attachment when compared to a hemostatic device secured to the right hand H1 or the forearm A of the patient using a seal member, etc. provided with an adhesive material.

Further, since the hemostatic device 100 includes the first inflatable portion 170 as a member that applies a compressive force to the puncture site t1, the compressive force can be easily adjusted by adjusting the internal pressure of the first inflatable portion 170. Further, in the hemostatic device 100, even when the first inflatable portion 170 is deformed to change the internal pressure following movement of the right hand H1 when the patient moves the right hand H1, the deformable second inflatable portion 180 mitigates the change in the internal pressure of the first inflatable portion 170. Therefore, the first inflatable portion 170 has a high following property to movement of the right hand H1 of the patient, and compression on the puncture site t1 by the first inflatable portion 170 can be appropriately maintained.

Further, the hemostatic device 100 is configured to cover only a part of the right hand H1 of the patient by the covering member 110, and is not configured to cover the entire right hand H1. For this reason, when the patient moves the right hand H1 in a state where the hemostatic device 100 is attached to the right hand H1 of the patient, it is possible to prevent movement of the right hand H1 of the patient from being transmitted to the entire hemostatic device 100. Therefore, the hemostatic device 100 can suppress position shift from the right hand H1 of the patient when the patient moves the right hand H1 in the state where the hemostatic device 100 is attached to the right hand H1 of the patient.

The hemostatic device 100 can be attached to the left hand H2 of the patient, for example, without changing the configuration of the hemostatic device 100. Hereinafter, a description will be given of an example of a procedure for attaching the hemostatic device 100 to the left hand H2 of the patient on which the puncture site t1 is formed. Note that description of content overlapping with the above-mentioned example of the procedure for attaching the hemostatic device 100 to the right hand H1 will be omitted.

As illustrated in Fig. 7A, the operator, etc. disposes the main body 120 of the covering member 110 so as to overlap the side of the dorsal side Hb of the right hand H2.

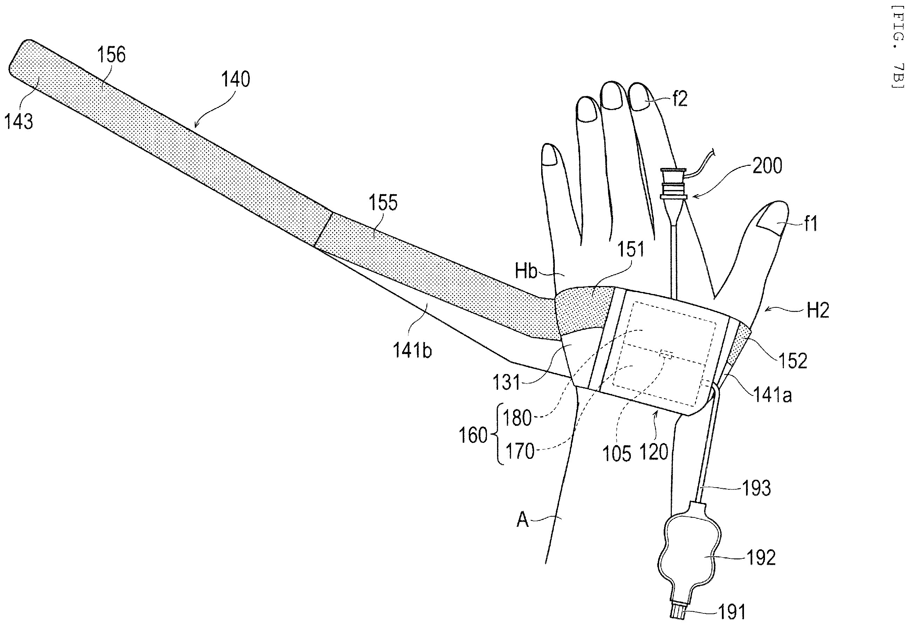

Subsequently, as illustrated in Fig. 7B, the operator, etc. wraps the inclined portion (the first inclined portion 141a and the second inclined portion 141b) 141 of the second arm portion 140 along the left hand H2 of the patient while wrapping the convex portion 133a of the first arm portion 130 around the left hand H2 of the patient. In this instance, the operator, etc. secures the convex portion 133a and the second arm portion 140 via the respective securing members 151 and 155 by bringing the fifth securing member 155 (female side of the surface fastener) disposed on the inner surface of the inclined portion 141 into contact with the first securing member 151 (male side of the surface fastener) disposed on the outer surface of the convex portion 133a of the first arm portion 130 on the palm Hp side of the left hand H2 of the patient.

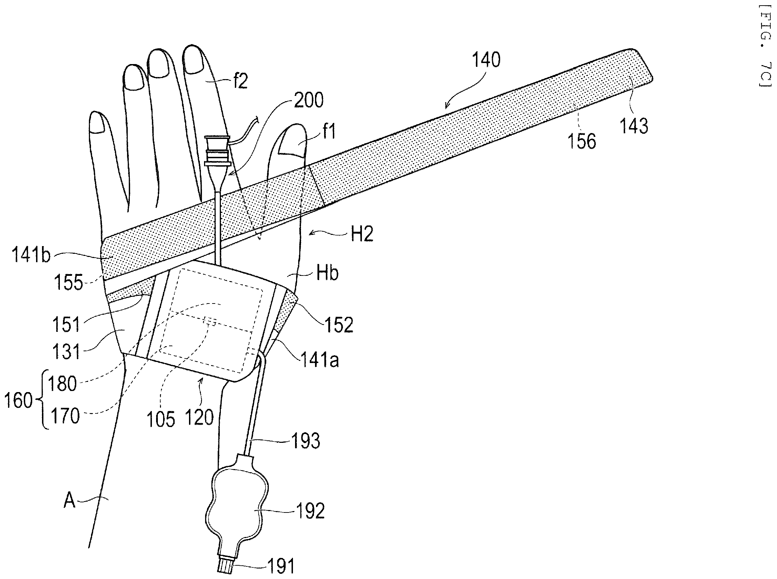

Subsequently, as illustrated in Fig. 7C, the operator, etc. disposes the second arm portion 140 on the side of the dorsal side Hb of the left hand H2 of the patient. In this instance, the operator, etc. secures the first arm portion 130 and the second arm portion 140 via the respective securing members 151 and 155 on the side of the dorsal side Hb of the left hand H2 by bringing the fifth securing member 155 (female side of the surface fastener) disposed on the inner surface of the inclined portion 141 into contact with the first securing member 151 (male side of the surface fastener) disposed on the outer surface of the inclined portion 131 of the first arm portion 130 on the side of the dorsal side Hb of the left hand H2 of the patient.

Subsequently, as illustrated in Fig. 7D, the operator, etc. passes the second arm portion 140 between the thumb f1 and the index finger f2 of the left hand H2 of the patient, and disposes the second arm end portion 143 on the palm Hp side of the left hand H2 of the patient. In this instance, the operator, etc. secures the first inclined portion 141a and the second arm end portion 143 via the respective securing members 152 and 156 by bringing the sixth securing member 156 (female side of the surface fastener) disposed on the inner surface of the second arm end portion 143 into contact with the second securing member 152 (male side of the surface fastener) disposed on the outer surface of the inclined portion 141 on the palm Hp side of the left hand H2 of the patient.

The operator, etc. can secure the hemostatic device 100 to the left hand H2 of the patient by the above procedure.

Hereinafter, the effects of the present embodiment will be described.

The hemostatic device 100 according to the present embodiment includes the covering member 110 disposed to cover the puncture site t1 on the right hand H1 (or left hand H2) of the patient, the plurality of securing members 151, 152, 153, 154, 155, and 156 that is configured to secure the covering member 110 in a state where the covering member 110 covers the puncture site t1, and the inflatable member 160 connected to the covering member 110 and configured to be inflated by injection of a fluid. The covering member 110 has the main body 120 to which the inflatable member 160 is connected, the first arm portion 130 protruding from the main body 120, and the second arm portion 140 protruding from the main body 120 while forming an obtuse angle with the longitudinal direction of the first arm portion 130. Further, the first arm portion 130 has the convex portion 133a protruding in the width direction of the first arm portion 130 at the end portion 133 of the first arm portion 130. Furthermore, the second arm portion 140 is longer than the first arm portion 130 and is configured to be secured to the convex portion 133a and secured to the second arm portion 140 by passing between the fingers f1 and f2 of the patient in a state where the second arm portion 140 is wrapped around the limb of the patient.

In the hemostatic device 100 configured as described above, it is possible to secure the main body 120 in which the inflatable member 160 is disposed to the limb by disposing a part of the second arm portion 140 between the adjacent fingers f1 and f2 of the patient and connecting the convex portion 133a and the second arm portion 140 while wrapping the first arm portion 130 and the second arm portion 140 around the limb of the patient. The first arm portion 130 and the second arm portion 140 are fastened to the limb by connecting the convex portion 133a and the second arm portion 140, and the main body 120 is secured to the limb. Further, by disposing the second arm portion 140 between the adjacent fingers f1 and f2 of the patient, it is possible to suppress position shift of the inflatable member 160 disposed in the main body 120 with respect to the puncture site t1. Further, the hemostatic device 100 has a simple securing structure capable of tightening the inflatable member 160 to the hand H1 of the patient by connecting the two arm portions 130 and 140 protruding from the main body 120. For this reason, the hemostatic device 100 can reduce the number of arm portions installed for securing the inflatable member 160. Therefore, the hemostatic device 100 can suppress an increase in arm portions that may interfere with a medical device such as the introducer 200 when the hemostatic device 100 is attached to the hand H1 of the patient, and it is possible to easily remove the medical device from the puncture site t1 even after the hemostatic device 100 is attached. Further, a movable range of the right hand H1 (or the left hand H2) increases from the wrist side to the fingertip side of the right hand H1. In the hemostatic device 100, the first arm portion 130 and the second arm portion 140 extend from the fingertip side to the wrist side of the right hand H1 while forming an obtuse angle, so that the first arm portion 130 and the second arm portion 140 can be connected on the wrist side of the right hand H1 of the patient. Therefore, in the hemostatic device 100, the first arm portion 130 and the second arm portion 140 can be secured on the wrist side where the movable range is small, and thus the main body 120 can be secured to the limb while maintaining the movable range on the fingertip side of the right hand H1. Note that an outer circumference of the right hand H1 becomes larger from the wrist side to the fingertip side of the right hand H1 in a state where the right hand H1 is spread. Therefore, since the first arm portion 130 and the second arm portion 140 extend in a direction opposite to a direction in which the outer circumference of the right hand H1 becomes larger, the first arm portion 130 and the second arm portion 140 can be reliably connected on the wrist side of the right hand H1 in the state where the hemostatic device 100 is attached. Furthermore, in the hemostatic device 100, even in the case where the patient moves the hand H with the inflatable member 160 inflated, when the first arm portion 130 and the second arm portion 140 are secured in a state of being tightened to the limb of the patient, it is possible to prevent rising of the distal side (fingertip side) of the main body 120 to which the inflatable member 160 is connected, and to appropriately maintain the compressive force of the inflatable member 160 on the puncture site t1 formed on the dorsal side Hb of the hand H1 of the patient.

Further, the main body 120 has first regions 121a and 121b and the second region 122 in which the inflatable member 160 is disposed unlike the first regions 121a and 121b. The first region 121a is disposed between the second region 122 and the first arm portion 130, and the first region 121b is disposed between the second region 122 and the second arm portion 140. Further, the second region 122 includes the support member 125 having a higher rigidity than that of the first regions 121a and 121b. Therefore, the hemostatic device 100 can prevent the inflatable member 160 from rising from the dorsal side Hb of the hand H of the patient by the support member 125 while hemostasis is performed. As a result, the hemostatic device 100 can suitably apply a compressive force from the inflatable member 160 to the puncture site t1 while being attached to the right hand H1 of the patient.

Further, the support member 125 has the curved portion 125a formed on the distal side of the main body 120. For this reason, in the support member 125, when the inflatable member 160 is inflated, a direction in which the inflatable member 160 applies a compressive force to the right hand H1 of the patient is directed in an oblique direction toward the puncture site t1. For this reason, the hemostatic device 100 can more effectively apply a compressive force to the puncture site t1. Further, the curved portion 125a of the support member 125 directs the compressive force of the inflatable member 160 toward the center side of the support member 125 by wrapping the end portion of the inflatable member 160 on the distal side when the inflatable member 160 inflates, and thus the compressive force of the inflatable member 160 is inhibited from escaping to the outside of the support member 125. For this reason, the hemostatic device 100 can suppress a decrease in the compressive force to the puncture site t1 by the inflatable member 160, and thus can appropriately maintain the compressive force of the inflatable member 160 to the puncture site t1.

Further, the inflatable member 160 has the first inflatable portion 170 and the deformable auxiliary member 180 having a smaller outer shape than that of the first inflatable portion 170, and the auxiliary member 180 is disposed to overlap the first inflatable portion 170 on the distal side of the main body 120. In the hemostatic device 100 configured as described above, when the first inflatable portion 170 inflates, the second inflatable portion 180 inhibits the first inflatable portion 170 from rising from the body surface of the patient. Therefore, when the first inflatable portion 170 inflates, the first inflatable portion 170 can effectively apply the compressive force to the puncture site t1. Further, when the patient bends a finger (for example, the thumb f1) or the wrist downward (to the palm Hp side of the hand H), the hemostatic device 100 maintains a state in which the auxiliary member 180 is deformed by following movement of the finger or the wrist and the auxiliary member 180 applies the compressive force to the puncture site t1.

Further, the auxiliary member is the second inflatable portion 180 configured to be inflated by injection of a fluid, and the lumen 181 of the second inflatable portion 180 communicates with the lumen 171 of the first inflatable portion 170. Since the auxiliary member 180 includes the second inflatable portion 180 which is inflatable, it is possible to improve the following property of the second inflatable portion 180 to movement of the right hand H1. Further, since the lumen 171 of the first inflatable portion 170 and the lumen 181 of the second inflatable portion 180 communicate with each other, the first inflatable portion 170 and the second inflatable portion 180 can be easily inflated.

In addition, the second inflatable portion 180 is disposed to have bilateral symmetry with respect to the center line d3 of the first inflatable portion 170, and the securing members 151 and 152 for securing the second arm portion 140 are disposed on the outer surface of the first arm portion 130 and the outer surface of the second arm portion 140 opposing to each other with the main body 120 interposed therebetween. For this reason, when the hemostatic device 100 is attached to the right hand H1 of the patient, the operator, etc. can secure the second arm portion 140 to the convex portion 133a through the respective securing members 151 and 152 while disposing the first inflatable portion 170 on the puncture site t1 so that the puncture site t1 is located on the center line d3 of the auxiliary member 180. Similarly, when the hemostatic device 100 is attached to the left hand H2 of the patient, the operator, etc. can secure the second arm portion 140 to the convex portion 133a through the respective securing members 151 and 152 while disposing the first inflatable portion 170 on the puncture site t1 so that the puncture site t1 is located on the center line d3 of the auxiliary member 180. Therefore, the operator, etc. can attach the hemostatic device 100 to both the right hand H1 and the left hand H2 of the patient.

Further, the convex portion 133a protrudes toward the distal side of the main body 120 in a direction intersecting the extending direction of the first arm portion 130. Therefore, when the second arm portion 140 is secured to the convex portion 133a, the operator, etc. can confirm the position of the convex portion 133a disposed on the palm Hp side of the hand H and the shape of the convex portion 133a by the hand feel, etc., and thus the second arm portion 140 can be easily secured to the convex portion 133a.

Further, the second arm portion 140 has the inclined portion 141 and the second arm end portion 143 which is continuously formed with the inclined portion 141 and forms an end portion of the second arm portion 140, and the width of the inclined portion 141 is larger than the width of the second arm end portion 143. Therefore, when the second arm portion 140 is secured to the convex portion 133a, by wrapping the inclined portion 141 around the hand H of the patient, the operator, etc. can more reliably dispose the inclined portion 141 with respect to the convex portion 133a, and easily secure the second arm portion 140.

Further, the hemostatic device 100 has the injection portion 191 for injecting a fluid into the inflatable member 160. The cushioning member 192 having an inflatable space is disposed between the injection portion 191 and the inflatable member 160. In the hemostatic device 100, when the patient moves the right hand H1 while the hemostatic device 100 is attached to the patient, the inflatable member 160 (the first inflatable portion 170 and the second inflatable portion 180) that compresses the puncture site t1 on the right hand H1 is deformed. When the inflatable member 160 is deformed, if there is no escape place for air in the lumen of the inflatable member 160 (the lumen 171 of the first inflatable portion 170 and the lumen 181 of the second inflatable portion 180), deformation of the inflatable member 160 is hindered. For this reason, the patient has a limited movable range for the right hand H1. The cushioning member 192 included in the hemostatic device 100 allows air to move from the lumen of the inflatable member 160 to the cushioning member 192 when the patient moves the right hand H1. For this reason, the patient can prevent the movable range from being restricted by the inflatable member 160 when the right hand H1 is moved. Note that when the patient returns the right hand H1 from the deformed state to the original state, air moves from the cushioning member 192 to the inflatable member 160, and thus the compressive force can be effectively applied to the puncture site t1 from the inflatable member 160.

### (Modification)

Next, a description will be given of a modification of the hemostatic device according to the first embodiment described above. In description of the modification, detailed description of the configuration, etc. previously described in the first embodiment will be omitted. In addition, content not particularly described in the description of the modification can be regarded as the same as that in the first embodiment.

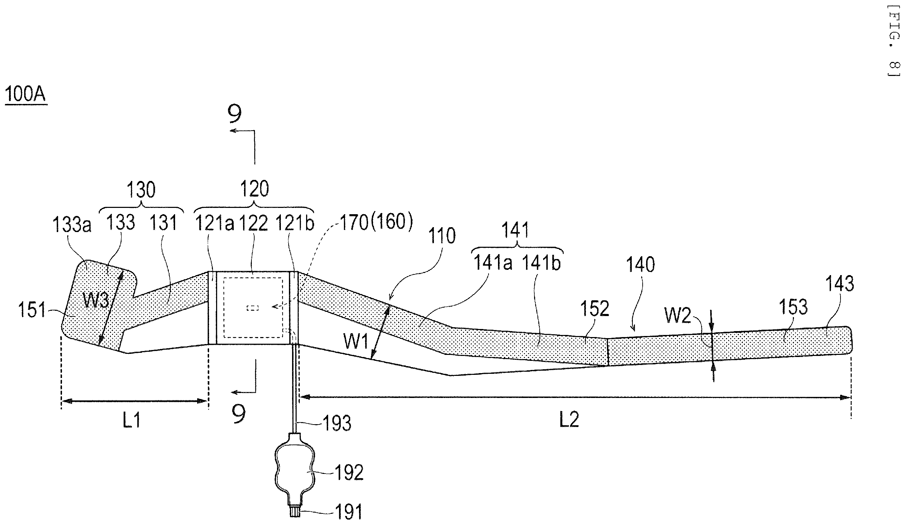

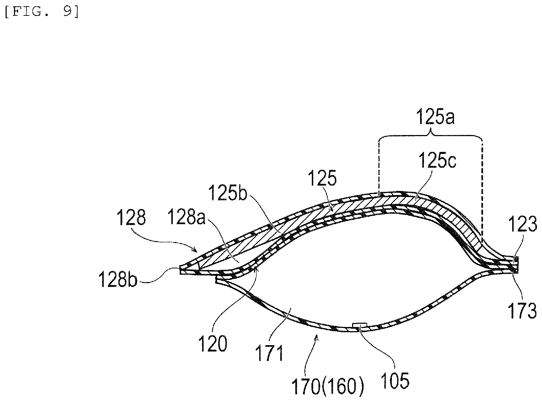

Fig. 8 is a plan view of a hemostatic device 100A seen from the outer surface side of the main body 120 of the covering member 110, and Fig. 9 is a cross-sectional view of the hemostatic device 10A taken along arrow 9-9 illustrated in Fig. 8.

In the hemostatic device 100A according to the modification, the inflatable member 160 includes only the first inflatable portion 170. That is, the inflatable member 160 does not include the second inflatable portion 180. Further, in the hemostatic device 100A, as illustrated in Fig. 9, the end portion 173 of the first inflatable portion 170 on the distal side included in the inflatable member 160 is directly fixed to the inner surface of the covering member 110. Other configurations of the hemostatic device 100A are substantially the same as those of the hemostatic device 100 described above.

In the hemostatic device 100A according to the modification, similarly to the hemostatic device 100 described above, position shift of the inflatable member 160 disposed on the main body 120 with respect to the puncture site t1 can be suppressed by connecting the convex portion 133a wrapped around the limb of the patient and the second arm portion 140, and securing a part of the second arm portion 140 passed between the fingers f1 and f2 of the patient to the second arm portion 140. Further, since the hemostatic device 100A has a simple securing structure capable of tightening the inflatable member 160 to the right hand H1 of the patient by connecting the two arm portions 130 and 140 protruding from the main body 120, the hemostatic device 100A can reduce the number of arm portions installed for securing the inflatable member 160. Therefore, the hemostatic device 100A can suppress an increase in arm portions that may interfere with a medical device such as the introducer 200 when the hemostatic device 100A is attached to the hand H1 of the patient, and it is possible to easily remove the medical device from the puncture site t1 even after the hemostatic device 100A is attached. Further, a movable range of the right hand H1 (or the left hand H2) of the patient increases from the wrist side to the fingertip side of the right hand H1. In the hemostatic device 100A, the first arm portion 130 and the second arm portion 140 extend from the fingertip side to the wrist side of the right hand H1 while forming an obtuse angle, so that the first arm portion 130 and the second arm portion 140 can be connected on the wrist side of the right hand H1 of the patient. Therefore, in the hemostatic device 100A, the first arm portion 130 and the second arm portion 140 can be secured on the wrist side where the movable range is small, and thus the main body 120 can be secured to the limb while maintaining the movable range on the fingertip side of the right hand H1. Note that the outer circumference of the right hand H1 becomes larger from the wrist side to the fingertip side of the right hand H1 in a state where the right hand H1 is spread. Therefore, since the first arm portion 130 and the second arm portion 140 extend in a direction opposite to a direction in which the outer circumference of the right hand H1 becomes larger, the first arm portion 130 and the second arm portion 140 can be reliably connected on the wrist side of the right hand H1 in the state where the hemostatic device 100A is attached. Furthermore, in the hemostatic device 100A, even in the case where the patient moves the right hand H1 with the inflatable member 160 inflated, when the first arm portion 130 and the second arm portion 140 are secured in a state of being tightened to the limb of the patient, it is possible to prevent rising of the distal side (fingertip side) of the main body 120 to which the inflatable member 160 is connected, and to appropriately maintain the compressive force of the inflatable member 160 on the puncture site t1 formed on the dorsal side Hb of the right hand H1 of the patient.

### (Second embodiment)

Next, a description will be given of a hemostatic device according to a second embodiment of the invention. In description of the second embodiment, detailed description of the configuration, etc. previously described in the first embodiment will be omitted. In addition, content not particularly described in the description of the second embodiment can be regarded as the same as that in the first embodiment.

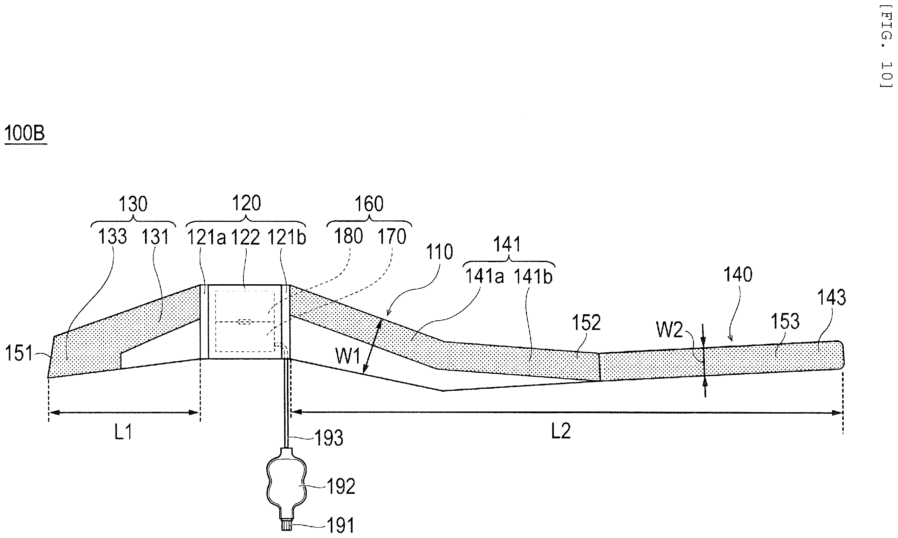

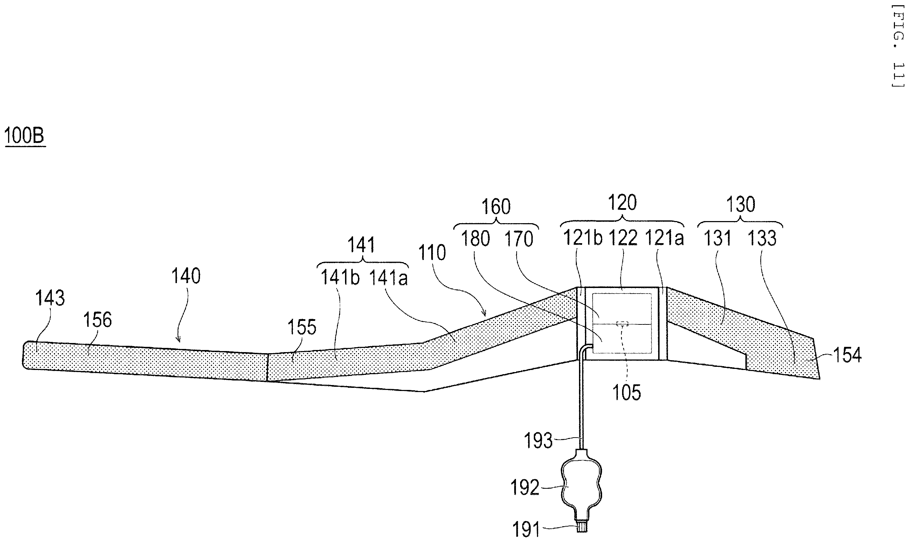

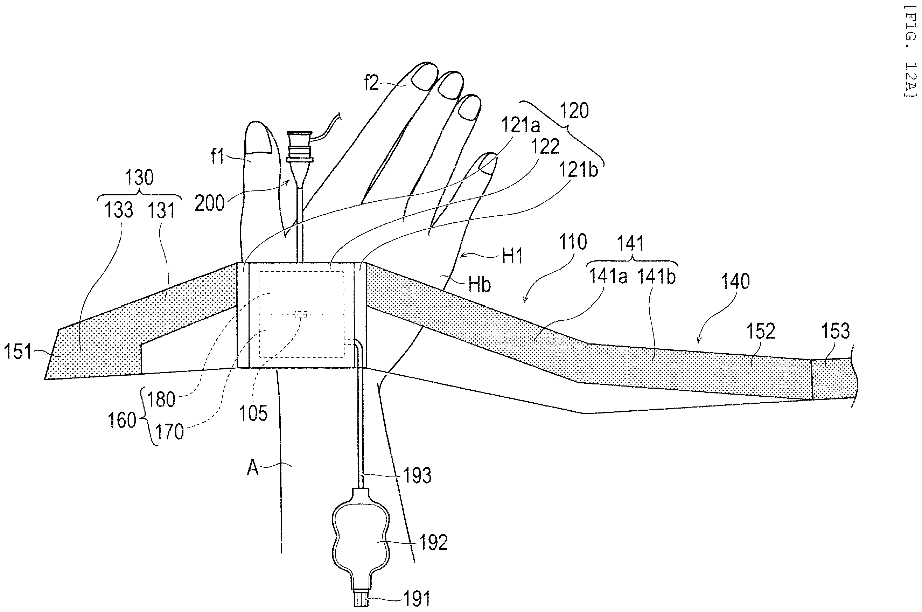

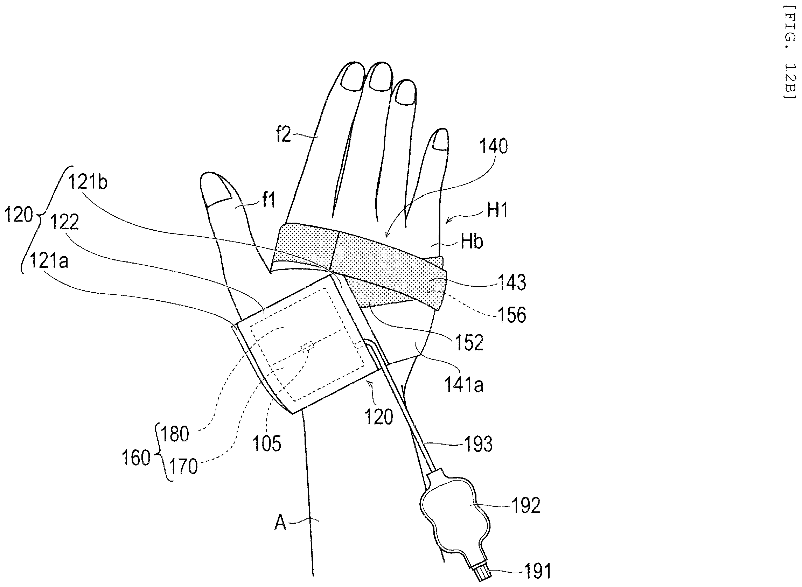

Fig. 10 is a plan view of a hemostatic device 100B seen from the outer surface side of the main body 120 of the covering member 110, and Fig. 11 is a plan view of the hemostatic device 100B seen from the inner surface side of the main body 120 of the covering member 110. Figs. 12A and 12B are diagrams briefly illustrating a usage example of the hemostatic device 100B.

As illustrated in Figs. 10 and 11, in the hemostatic device 100B according to the second embodiment, the first arm portion 130 does not include the convex portion 133a (see Figs. 1 and 2). In this respect, the second embodiment differs from the hemostatic device 100 according to the first embodiment.

The end portion 133 of the first arm portion 130 of the hemostatic device 100B is continuously connected to the inclined portion 131, and extends linearly along a direction substantially the same as the extending direction of the inclined portion 131.

As illustrated in Fig. 10, a first securing member 151 is disposed on a part of the outer surface of the first arm portion 130 on the distal side. Further, as illustrated in Fig. 11, a fourth securing member 154 is disposed on a part of the inner surface of the first arm portion 130 on the distal side.

Fig. 12A illustrates a state before the hemostatic device 100B is attached to the limb of the patient, and Fig. 12B illustrates a state in which the hemostatic device 100B is attached to the limb of the patient. The hemostatic device 100B can be attached to the right hand H1 or the left hand H2 of the patient on which the puncture site t1 is formed by substantially the same procedure as the attachment procedure of the hemostatic device 100 according to the first embodiment described above. The procedure for attaching the hemostatic device 100B to the right hand H1 of the patient will be outlined below.

As illustrated in Fig. 12A, the operator, etc. disposes the main body 120 of the covering member 110 so that the main body 120 overlaps the side of the dorsal side Hb of the right hand H1. Subsequently, the operator, etc. wraps the inclined portion (the first inclined portion 141a and the second inclined portion 141b) 141 of the second arm portion 140 along the right hand H1 of the patient while wrapping the end portion 133 of the first arm portion 130 around the right hand H1 of the patient. In this instance, the operator, etc. secures the end portion 133 and the second arm portion 140 via the respective securing members 151 and 155 by bringing the fifth securing member 155 (female side of the surface fastener) disposed on the inner surface of the inclined portion 141 into contact with the first securing member 151 (male side of the surface fastener) disposed on the outer surface of the end portion 133 of the first arm portion 130 on the palm Hp side of the right hand H1 of the patient.

Subsequently, the operator, etc. passes the second arm portion 140 between the thumb f1 and the index finger f2 of the right hand H1 of the patient, and disposes the second arm end portion 143 on the side of the dorsal side Hb of the right hand H1 of the patient. In this instance, the operator, etc. secures the first inclined portion 141a and the second arm end portion 143 via the respective securing members 152 and 156 by bringing the sixth securing member 156 (female side of the surface fastener) disposed on the inner surface of the second arm end portion 143 into contact with the second securing member 152 (male side of the surface fastener) disposed on the outer surface of the inclined portion 141 on the side of the dorsal side Hb of the right hand H1 of the patient.

By the above procedure, as illustrated in Fig. 12B, the operator, etc. can secure the hemostatic device 100 to the right hand H1 of the patient.

As described above, the hemostatic device 100B according to the second embodiment includes the covering member 110 disposed to cover the puncture site t1 on the right hand H1 (or left hand H2) of the patient, the plurality of securing members 151, 152, 153, 154, 155, and 156 that is configured to secure the covering member 110 in a state where the covering member 110 covers the puncture site t1, and the inflatable member 160 connected to the covering member 110 and configured to be inflated by injection of a fluid. Further, the covering member 110 has the main body 120 to which the inflatable member 160 is connected, the first arm portion 130 protruding from the main body 120, and the second arm portion 140 protruding from the main body 120 while forming an obtuse angle with the longitudinal direction of the first arm portion 130. Further, the inflatable member 160 has the first inflatable portion 170 and the deformable auxiliary member 180 which has a smaller outer shape than that of the first inflatable portion 170 and is biased toward the distal side (one end side) of the first inflatable portion 170. In the covering member 110, the first arm portion 130 and the second arm portion 140 can be connected by the securing members 151 and 155 in a state where the covering member 110 is wrapped around the limb of the patient, and the auxiliary member 180 is disposed between the first arm portion 130 and the second arm portion 140.

In the hemostatic device 100B configured as described above, since the first arm portion 130 and the second arm portion 140 protrude from the main body 120 so as to form an obtuse angle, when the hemostatic device 100B is attached to the right hand H1 of the patient, the arm portions 130 and 140 can be connected so that the entire finger of the patient is not covered by the covering member 110. For this reason, the patient is less likely to be restricted in movement of the right hand H1 even when the hemostatic device 100B is attached, and thus a degree of freedom on the fingertip side can be increased. Further, since the hemostatic device 100B has the deformable auxiliary member 180 located on the distal side of the first inflatable portion 170, when the first inflatable portion 170 is inflated, the auxiliary member 180 presses the first inflatable portion 170 against the limb of the patient to inhibit the first inflatable portion 170 from rising in a direction away from the puncture site t1 on the right hand H1 of the patient. As a result, the hemostatic device 100B can maintain an appropriate compressive force on the puncture site t1 while increasing the degree of freedom on the fingertip side. Furthermore, in the hemostatic device 100B, since the first arm portion 130 and the second arm portion 140 extend from the fingertip side to the wrist side of the right hand H1 while forming an obtuse angle, the first arm portion 130 and the second arm portion 140 can be secured on the wrist side where the movable range is small. Therefore, the main body 120 can be appropriately secured to the limb while maintaining the movable range on the fingertip side of the right hand H1. Further, in the hemostatic device 100B, since the first inflatable portion 170 can be secured to the puncture site t1 by the two arm portions including the first arm portion 130 and the second arm portion 140 and the auxiliary member 180, it is unnecessary to add different arm portions between the first arm portion 130 and the second arm portion 140. Therefore, the operator, etc. can easily remove the medical device such as the introducer 200 after attaching the hemostatic device 100B. Further, since the first arm portion 130 and the second arm portion 140 are connected in a state of being wrapped around the right hand H1 of the patient, the first arm portion 130 and the second arm portion 140 are pressed against the right hand H1 of the patient while pulling the main body 120 located therebetween toward the both side portion sides (vertical direction in Fig. 10). Since the auxiliary member 180 is located between the first arm portion 130 and the second arm portion 140, the inflatable member 160 can be reliably pressed against the body surface of the right hand H1 by connection of the first arm portion 130 and the second arm portion 140. Therefore, the hemostatic device 100B can press the first inflatable portion 170 against the limb of the patient by the auxiliary member 180, and reliably inhibit the first inflatable portion 170 from rising in the direction away from the puncture site t1 on the right hand H1 of the patient. In this way, the hemostatic device 100B can reliably press the first inflatable portion 170 against the right hand H1 of the patient and maintain an appropriate compressive force on the puncture site t1 while increasing the degree of freedom on the fingertip side during attachment of the hemostatic device 100B.

Further, the main body 120 has the first regions 121a and 121b and the second region 122 in which the inflatable member 160 is disposed unlike the first regions 121a and 121b. The first region 121a is disposed between the second region 122 and the first arm portion 130, and the first region 121b is disposed between the second region 122 and the second arm portion 140. Further, the second region 122 includes the support member 125 having a higher rigidity than that of the first regions 121a and 121b. Therefore, the hemostatic device 100B can prevent the inflatable member 160 from rising from the dorsal side Hb of the hand H of the patient by the support member 125 while hemostasis is performed. As a result, the hemostatic device 100 can suitably apply a compressive force from the inflatable member 160 to the puncture site t1 while being attached to the hand H of the patient.

Further, the support member 125 has the curved portion 125a formed on the distal side of the main body 120. For this reason, in the support member 125, when the inflatable member 160 is inflated, a direction in which the inflatable member 160 applies a compressive force to the right hand H1 of the patient is directed in an oblique direction toward the puncture site t1. For this reason, the hemostatic device 100 can more effectively apply a compressive force to the puncture site t1. Further, when the curved portion 125a of the support member 125 wraps the end portion of the inflatable member 160 on the distal side, the inflatable member 160 directs the compressive force toward the central side of the support member 125, and thus the compressive force of the second inflatable portion 180 is inhibited from escaping to the outside of the support member 125. In this way, even in the case where a gap is generated between the first arm portion 130 and the right hand H1 of the patient, when the curved portion 125a presses the second inflatable portion 180 against the skin of the right hand H1 of the patient, it is possible to prevent the first inflatable portion 170 from rising, and it is possible to prevent a decrease in the compressive force applied to the puncture site t1 by the inflatable member 160.

Further, the auxiliary member is the second inflatable portion 180 configured to be inflated by injection of a fluid, and the lumen 181 of the second inflatable portion 180 communicates with the lumen 171 of the first inflatable portion 170. Since the auxiliary member 180 includes the second inflatable portion 180 which is inflatable, it is possible to improve the following property of the second inflatable portion 180 to movement of the right hand H1. Further, since the lumen 171 of the first inflatable portion 170 and the lumen 181 of the second inflatable portion 180 communicate with each other, the first inflatable portion 170 and the second inflatable portion 180 can be easily inflated.

Further, the second arm portion 140 is longer than the first arm portion 130, and is configured to be secured to the second arm portion 140 by passing between the fingers f1 and f2 of the patient in a state where the second arm portion 140 is wrapped around the limb of the patient. For this reason, the hemostatic device 100B can secure the main body 120 in which the inflatable member 160 is disposed to the limb by disposing a part of the second arm portion 140 between the adjacent fingers f1 and f2 of the patient and connecting parts of the second arm portion 140 to each other while wrapping the first arm portion 130 and the second arm portion 140 around the limb of the patient. The first arm portion 130 and the second arm portion 140 are fastened to the limb by connecting the parts of the second arm portions 140 to each other, and the main body 120 is secured to the limb. By disposing the second arm portion 140 between the adjacent fingers f1 and f2 of the patient, it is possible to effectively suppress position shift of the inflatable member 160 disposed on the main body 120 with respect to the puncture site t1.

In addition, the second inflatable portion 180 is disposed to have bilateral symmetry with respect to the center line d3 of the first inflatable portion 170, and the securing members 151 and 152 for securing the second arm portion 140 are disposed on the outer surfaces of the first arm portion 130 and the second arm portion 140 opposing to each other with the main body 120 interposed therebetween. For this reason, when the hemostatic device 100 is attached to the right hand H1 of the patient, the operator, etc. can secure the respective arm portions 130 and 140 through the respective securing members 151 and 152 while disposing the first inflatable portion 170 on the puncture site t1 so that the puncture site t1 is located on the center line d3 of the auxiliary member 180. Similarly, when the hemostatic device 100 is attached to the left hand H2 of the patient, the operator, etc. can secure the respective arm portions 130 and 140 through the respective securing members 151 and 152 while disposing the first inflatable portion 170 on the puncture site t1 so that the puncture site t1 is located on the center line d3 of the auxiliary member 180. Therefore, the operator, etc. can attach the hemostatic device 100 to both the right hand H1 and the left hand H2 of the patient.

Further, the second arm portion 140 has the inclined portion 141 and the second arm end portion 143 which is continuously formed with the inclined portion 141 and forms an end portion of the second arm portion 140, and the width of the inclined portion 141 is larger than the width of the second arm end portion 143. Therefore, when the inclined portion 141 is wrapped around the hand H of the patient, the operator, etc. can more reliably dispose the inclined portion 141 on the second arm end portion 143, and easily secure the second arm portion 140.

Further, the hemostatic device 100B has the injection portion 191 for injecting a fluid into the inflatable member 160. The cushioning member 192 having an inflatable space is disposed between the injection portion 191 and the inflatable member 160. In the hemostatic device 100B, when the patient moves the right hand H1 while the hemostatic device 100B is attached to the patient, the inflatable member 160 (the first inflatable portion 170 and the second inflatable portion 180) that compresses the puncture site t1 on the right hand H1 is deformed. When the inflatable member 160 is deformed, if there is no escape place for air in the lumen of the inflatable member 160 (the lumen 171 of the first inflatable portion 170 and the lumen 181 of the second inflatable portion 180), deformation of the inflatable member 160 is hindered. For this reason, the patient has a limited movable range for the right hand H1. The cushioning member 192 included in the hemostatic device 100B allows air to move from the lumen of the inflatable member 160 to the cushioning member 192 when the patient moves the right hand H1. For this reason, the patient can prevent the movable range from being restricted by the inflatable member 160 when the right hand H1 is moved. Note that when the patient returns the right hand H1 from the deformed state to the original state, air moves from the cushioning member 192 to the inflatable member 160, and thus the compressive force can be effectively applied to the puncture site t1 from the inflatable member 160.

Even though the hemostatic device according to the invention has been described above through the embodiments, the invention is not limited to content described in this specification, and can be appropriately modified based on description of the scope of claims.

The auxiliary member is not limited to the inflatable member described in each embodiment. For example, the auxiliary member may include a member made of a resin material such as plastic, gel, etc., a member containing gel whose moisture content decreases over time to gradually reduce a compressive force, an elastic material such as a sponge-like substance, an aggregate of fibers such as cotton, metal, a member having a predetermined three-dimensional shape (sphere, ellipsoid, triangular pyramid, etc.), an appropriate combination thereof, etc.

In addition, the shape and dimensions of each portion of the hemostatic device are not particularly limited as long as the inflatable member can be disposed at the puncture site while wrapping the first arm portion and the second arm portion around the limb, and changes can be made as appropriate.

This application is based on Japanese Patent Application No. 2018-145366 filed on August 1, 2018.

### Reference Signs List

- 100, 100A, 100B: hemostatic device,
- 105: marker portion,
- 110: covering member,
- 120: main body,
- 121a, 121b: first region,
- 122: second region,
- 125: support member,
- 125a: curved portion,
- 128: cover member,
- 128a: insertion portion,
- 130: first arm portion,
- 131: inclined portion,
- 133: end portion,
- 133a: convex portion,
- 140: second arm portion,
- 141: inclined portion,
- 141a: first inclined portion,
- 141b: second inclined portion,
- 143: second arm end portion,
- 151: first securing member (securing member),
- 152: second securing member (securing member),
- 153: third securing member (securing member),
- 154: fourth securing member (securing member),
- 155: fifth securing member (securing member),
- 156: sixth securing member (securing member),
- 160: inflatable member,
- 170: first inflatable portion,
- 171: lumen of first inflatable portion,
- 180: second inflatable portion (auxiliary member),
- 181: lumen of second inflatable portion,
- 191: injection portion,
- 192: cushioning member,
- 200: introducer,
- A: forearm,
- B1: palmar artery,
- H1: right hand (hand),
- H2: left hand (hand),
- Hb: dorsal side of hand,
- Hp: palm of hand,
- f1: thumb (finger),
- f2: index finger (finger),
- t1: puncture site (site where bleeding is to be stopped)

## Claims

1. A hemostatic device (100, 100A, 100B) comprising:
a covering member (110) configured to be disposed to cover a site (t1) where bleeding is to be stopped on a hand (H) of a patient;
a securing member (151, 152, 153, 154, 155, 156) configured to secure the covering member (110) in a state where the covering member (110) covers the site (t1) where bleeding is to be stopped; and
an inflatable member (160) connected to the covering member (110) and configured to be inflated by injection of a fluid,
wherein the covering member (110) includes a main body (120) to which the inflatable member (160) is connected, a first arm portion (130) protruding from the main body (120), and a second arm portion (140) protruding from the main body (120) while forming an obtuse angle (θ1) with a longitudinal direction of the first arm portion (130),
the first arm portion (130) has a convex portion (133a) protruding in a width direction of the first arm portion (130) at an end portion (133) of the first arm portion (130), and
the second arm portion (140) is longer than the first arm portion (130) and is configured to be secured to the convex portion (133a) and secured to the second arm portion (140) by passing between fingers of the patient in a state where the second arm portion (140) is wrapped around a limb of the patient,
**characterized in that**
the inflatable member (160) has a first inflatable portion (170) and a deformable auxiliary member (180) having a smaller outer shape than an outer shape of the first inflatable portion (170), and
the auxiliary member (180) is disposed to overlap the first inflatable portion (170) on a distal side of the main body (120).

2. The hemostatic device (100, 100A, 100B) according to claim 1,
wherein the main body (120) has a first region (121a, 121b) and a second region (122) in which the inflatable member (160) is disposed unlike the first region (121a, 121b),
the first region (121a, 121b) is disposed between the second region (122) and the first arm portion (130) and between the second region (122) and the second arm portion (140), and
the second region (122) has a support member (125) having a higher rigidity than a rigidity of the first region (121a, 121b).

3. The hemostatic device (100, 100A, 100B) according to claim 2, wherein the support member (125) has a curved portion (125a) formed on a distal side of the main body (120).

4. The hemostatic device (100, 100A, 100B) according to any one of claims 1 to 3,
wherein the auxiliary member (180) is a second inflatable portion (180) configured to be inflated by injection of a fluid, and
a lumen (181) of the second inflatable portion (180) communicates with a lumen (171) of the first inflatable portion (170).

5. The hemostatic device (100, 100A, 100B) according to any one of claims 1 to 4,
wherein the auxiliary member (180) is disposed to have bilateral symmetry with respect to a center line of the first inflatable portion (170), and
the securing member (151, 152, 153, 154, 155, 156) for securing the second arm portion (140) is disposed on outer surfaces of the first arm portion (130) and the second arm portion (140) opposing to each other with the main body (120) interposed therebetween.

6. The hemostatic device (100, 100A, 100B) according to any one of claims 1 to 5, wherein the convex portion (133a) protrudes in a direction intersecting an extending direction of the first arm portion (130) toward a distal side of the main body (120).

7. The hemostatic device (100, 100A, 100B) according to any one of claims 1 to 6,
wherein the second arm portion (140) has an inclined portion (141) and a second arm end portion (143) which is continuously formed with the inclined portion (141) and forms an end portion of the second arm portion (140), and
a width of the inclined portion (141) is larger than a width of the second arm end portion (143).

8. The hemostatic device (100, 100A, 100B) according to any one of claims 1 to 7,
wherein an injection portion (191) for injecting a fluid is provided to the inflatable member (160), and
a cushioning member (192) having an inflatable space is disposed between the injection portion (191) and the inflatable member (160).

9. A hemostatic device (100, 100A, 100B) comprising:
a covering member (110) configured to be disposed to cover a site (t1) where bleeding is to be stopped on a hand (H) of a patient;
a securing member (151, 152, 153, 154, 155, 156) configured to secure the covering member (110) in a state where the covering member (110) covers the site (t1) where bleeding is to be stopped; and
an inflatable member (160) connected to the covering member (110) and configured to be inflated by injection of a fluid,
wherein the covering member (110) includes a main body (120) to which the inflatable member (160) is connected, a first arm portion (130) protruding from the main body (120), and a second arm portion (140) protruding from the main body (120) while forming an obtuse angle with a longitudinal direction of the first arm portion (130),
the first arm portion (130) and the second arm portion (140) of the covering member (110) are configured to be connectable by the securing member (151, 152, 153, 154, 155, 156) in a state where the covering member (110) is wrapped around a limb of the patient, and
**characterized in that** the inflatable member (160) has a first inflatable portion (170) and a deformable auxiliary member (180) which has a smaller outer shape than an outer shape of the first inflatable portion (170) and is biased toward one end side of the first inflatable portion (170) and **in that** the auxiliary member (180) is disposed between the first arm portion (130) and the second arm portion (140).

10. The hemostatic device (100, 100A, 100B)according to claim 9,
wherein the main body (120) has a first region (121a, 121b) and a second region (122) in which the inflatable member (160) is disposed unlike the first region (121a, 121b),
the first region (121a, 121b) is disposed between the second region (122) and the first arm portion (130) and between the second region (122) and the second arm portion (140), and
the second region (122) has a support member (125) having a higher rigidity than a rigidity of the first region (121a, 121b).

11. The hemostatic device (100, 100A, 100B) according to claim 10, wherein the support member (125) has a curved portion (125a) formed on a distal side of the main body (120).

12. The hemostatic device (100, 100A, 100B) according to any one of claims 9 to 11,
wherein the auxiliary member (180) is a second inflatable portion (180) configured to be inflated by injection of a fluid, and
a lumen (181) of the second inflatable portion (180) communicates with a lumen (171) of the first inflatable portion (170).

13. The hemostatic device (100, 100A, 100B) according to any one of claims 9 to 12, wherein the second arm portion (140) is longer than the first arm portion (130) and is configured to be secured to the second arm portion (140) by passing between fingers of the patient in a state where the second arm portion (140) is wrapped around the limb of the patient.

14. The hemostatic device (100, 100A, 100B) according to any one of claims 9 to 13,
wherein the auxiliary member (180) is disposed to have bilateral symmetry with respect to a center line of the first inflatable portion (170), and
the securing member (151, 152, 153, 154, 155, 156) for securing the second arm portion (140) is disposed on outer surfaces of the first arm portion (130) and the second arm portion (140) opposing to each other with the main body (120) interposed therebetween.

15. The hemostatic device (100, 100A, 100B) according to any one of claims 9 to 14,
wherein the second arm portion (140) has an inclined portion (141) and a second arm end portion (143) which is continuously formed with the inclined portion (141) and forms an end portion of the second arm portion (140), and
a width of the inclined portion (141) is larger than a width of the second arm end portion (140).

16. The hemostatic device (100, 100A, 100B) according to any one of claims 9 to 15,
wherein an injection portion (191) for injecting a fluid is provided to the inflatable member (160), and
a cushioning member (192) having an inflatable space is disposed between the injection portion (191) and the inflatable member (160).

## Patentansprüche

1. Hämostatische Vorrichtung (100, 100A, 100B), umfassend:
ein Abdeckelement (110), das so konfiguriert ist, dass es so angeordnet werden kann, dass es eine Stelle (t1) an einer Hand (H) eines Patienten abdeckt, an welcher die Blutung gestoppt werden soll;
ein Sicherungselement (151, 152, 153, 154, 155, 156), das so konfiguriert ist, dass es das Abdeckelement (110) in einem Zustand sichert, in dem das Abdeckelement (110) die Stelle (t1) abdeckt, an welcher die Blutung gestoppt werden soll; und
ein aufblasbares Element (160), das mit dem Abdeckelement (110) verbunden und so konfiguriert ist, dass es durch Injektion eines Fluids aufgeblasen werden kann,
wobei das Abdeckelement (110) einen Hauptkörper (120), mit dem das aufblasbare Element (160) verbunden ist, einen ersten Armabschnitt (130), der von dem Hauptkörper (120) hervorsteht, und einen zweiten Armabschnitt (140), der von dem Hauptkörper (120) hervorsteht, aufweist, während er einen stumpfen Winkel (θ1) mit einer Längsrichtung des ersten Armabschnitts (130) bildet,
der erste Armabschnitt (130) einen konvexen Abschnitt (133a) aufweist, der in einer Breitenrichtung des ersten Armabschnitts (130) an einem Endabschnitt (133) des ersten Armabschnitts (130) hervorsteht, und
der zweite Armabschnitt (140) länger ist als der erste Armabschnitt (130) und so konfiguriert ist, dass er an dem konvexen Abschnitt (133a) gesichert werden kann und an dem zweiten Armabschnitt (140) gesichert werden kann, indem er zwischen den Fingern des Patienten in einem Zustand hindurchgeführt wird, in dem der zweite Armabschnitt (140) um eine Gliedmaße des Patienten gewickelt ist,
**dadurch gekennzeichnet, dass**
das aufblasbare Element (160) einen ersten aufblasbaren Abschnitt (170) und ein verformbares Hilfselement (180) aufweist, das eine kleinere äußere Form als eine äußere Form des ersten aufblasbaren Abschnitts (170) aufweist, und
das Hilfselement (180) so angeordnet ist, dass es den ersten aufblasbaren Abschnitt (170) an einer distalen Seite des Hauptkörpers (120) überlappt.

2. Hämostatische Vorrichtung (100, 100A, 100B) nach Anspruch 1,
wobei der Hauptkörper (120) einen ersten Bereich (121a, 121b) und einen zweiten Bereich (122) aufweist, in dem das aufblasbare Element (160) im Gegensatz zu dem ersten Bereich (121a, 121b) angeordnet ist,
der erste Bereich (121a, 121b) zwischen dem zweiten Bereich (122) und dem ersten Armabschnitt (130) und zwischen dem zweiten Bereich (122) und dem zweiten Armabschnitt (140) angeordnet ist, und
der zweite Bereich (122) ein Stützelement (125) aufweist, das eine höhere Steifigkeit als eine Steifigkeit des ersten Bereichs (121a, 121b) aufweist.

3. Hämostatische Vorrichtung (100, 100A, 100B) nach Anspruch 2, wobei das Stützelement (125) einen gekrümmten Abschnitt (125a) aufweist, der an einer distalen Seite des Hauptkörpers (120) ausgebildet ist.

4. Hämostatische Vorrichtung (100, 100A, 100B) nach einem der Ansprüche 1 bis 3,
wobei das Hilfselement (180) ein zweiter aufblasbarer Abschnitt (180) ist, der so konfiguriert ist, dass er durch Injektion eines Fluids aufgeblasen wird, und
ein Lumen (181) des zweiten aufblasbaren Abschnitts (180) mit einem Lumen (171) des ersten aufblasbaren Abschnitts (170) in Verbindung steht.

5. Hämostatische Vorrichtung (100, 100A, 100B) nach einem der Ansprüche 1 bis 4,
wobei das Hilfselement (180) so angeordnet ist, dass es eine bilaterale Symmetrie in Bezug auf eine Mittellinie des ersten aufblasbaren Abschnitts (170) aufweist, und
das Sicherungselement (151, 152, 153, 154, 155, 156) zum Sichern des zweiten Armabschnitts (140) an Außenflächen des ersten Armabschnitts (130) und des zweiten Armabschnitts (140), die einander gegenüberliegen, angeordnet ist, wobei der Hauptkörper (120) dort dazwischen angeordnet ist.

6. Hämostatische Vorrichtung (100, 100A, 100B) nach einem der Ansprüche 1 bis 5, wobei der konvexe Abschnitt (133a) in einer Richtung, die eine Erstreckungsrichtung des ersten Armabschnitts (130) schneidet, in Richtung einer distalen Seite des Hauptkörpers (120) hervorsteht.

7. Hämostatische Vorrichtung (100, 100A, 100B) nach einem der Ansprüche 1 bis 6,
wobei der zweite Armabschnitt (140) einen geneigten Abschnitt (141) und einen zweiten Armendabschnitt (143) aufweist, der kontinuierlich mit dem geneigten Abschnitt (141) ausgebildet ist und einen Endabschnitt des zweiten Armabschnitts (140) bildet, und
eine Breite des geneigten Abschnitts (141) größer ist als eine Breite des zweiten Armendabschnitts (143).

8. Hämostatische Vorrichtung (100, 100A, 100B) nach einem der Ansprüche 1 bis 7,
wobei ein Injektionsabschnitt (191) zum Injizieren eines Fluids an dem aufblasbaren Element (160) vorgesehen ist, und
ein Polsterungselement (192), das einen aufblasbaren Raum aufweist, zwischen dem Injektionsabschnitt (191) und dem aufblasbaren Element (160) angeordnet ist.

9. Hämostatische Vorrichtung (100, 100A, 100B), umfassend:
ein Abdeckelement (110), das so konfiguriert ist, dass es so angeordnet werden kann, dass es eine Stelle (t1) an einer Hand (H) eines Patienten abdeckt, an welcher die Blutung gestoppt werden soll;
ein Sicherungselement (151, 152, 153, 154, 155, 156), das so konfiguriert ist, dass es das Abdeckelement (110) in einem Zustand sichert, in dem das Abdeckelement (110) die Stelle (t1) abdeckt, an welcher die Blutung gestoppt werden soll; und
ein aufblasbares Element (160), das mit dem Abdeckelement (110) verbunden und so konfiguriert ist, dass es durch Injektion eines Fluids aufgeblasen werden kann,
wobei das Abdeckelement (110) einen Hauptkörper (120), mit dem das aufblasbare Element (160) verbunden ist, einen ersten Armabschnitt (130), der von dem Hauptkörper (120) hervorsteht, und einen zweiten Armabschnitt (140), der von dem Hauptkörper (120) hervorsteht, aufweist, während er einen stumpfen Winkel mit einer Längsrichtung des ersten Armabschnitts (130) bildet,
der erste Armabschnitt (130) und der zweite Armabschnitt (140) des Abdeckelements (110) so konfiguriert sind, dass sie in einem Zustand, in dem das Abdeckelement (110) um eine Gliedmaße des Patienten gewickelt ist, durch das Sicherungselement (151, 152, 153, 154, 155, 156) verbunden werden können, und
**dadurch gekennzeichnet, dass** das aufblasbare Element (160) einen ersten aufblasbaren Abschnitt (170) und ein verformbares Hilfselement (180) aufweist, das eine kleinere äußere Form als eine äußere Form des ersten aufblasbaren Abschnitts (170) aufweist und in Richtung einer Endseite des ersten aufblasbaren Abschnitts (170) vorgespannt ist, und dadurch dass
das Hilfselement (180) zwischen dem ersten Armabschnitt (130) und dem zweiten Armabschnitt (140) angeordnet ist.

10. Hämostatische Vorrichtung (100, 100A, 100B) nach Anspruch 9,
wobei der Hauptkörper (120) einen ersten Bereich (121a, 121b) und einen zweiten Bereich (122) aufweist, in dem das aufblasbare Element (160) im Gegensatz zu dem ersten Bereich (121a, 121b) angeordnet ist,
der erste Bereich (121a, 121b) zwischen dem zweiten Bereich (122) und dem ersten Armabschnitt (130) und zwischen dem zweiten Bereich (122) und dem zweiten Armabschnitt (140) angeordnet ist, und
der zweite Bereich (122) ein Stützelement (125) aufweist, das eine höhere Steifigkeit als eine Steifigkeit des ersten Bereichs (121a, 121b) aufweist.

11. Hämostatische Vorrichtung (100, 100A, 100B) nach Anspruch 10, wobei das Stützelement (125) einen gekrümmten Abschnitt (125a) aufweist, der an einer distalen Seite des Hauptkörpers (120) ausgebildet ist.

12. Hämostatische Vorrichtung (100, 100A, 100B) nach einem der Ansprüche 9 bis 11,
wobei das Hilfselement (180) ein zweiter aufblasbarer Abschnitt (180) ist, der so konfiguriert ist, dass er durch Injektion eines Fluids aufgeblasen wird, und
ein Lumen (181) des zweiten aufblasbaren Abschnitts (180) mit einem Lumen (171) des ersten aufblasbaren Abschnitts (170) in Verbindung steht.

13. Hämostatische Vorrichtung (100, 100A, 100B) nach einem der Ansprüche 9 bis 12, wobei der zweite Armabschnitt (140) länger ist als der erste Armabschnitt (130) und so konfiguriert ist, dass er an dem zweiten Armabschnitt (140) gesichert werden kann, indem er zwischen den Fingern des Patienten in einem Zustand hindurchgeführt wird, in dem der zweite Armabschnitt (140) um die Gliedmaße des Patienten gewickelt ist.

14. Hämostatische Vorrichtung (100, 100A, 100B) nach einem der Ansprüche 9 bis 13,
wobei das Hilfselement (180) so angeordnet ist, dass es eine bilaterale Symmetrie in Bezug auf eine Mittellinie des ersten aufblasbaren Abschnitts (170) aufweist, und
das Sicherungselement (151, 152, 153, 154, 155, 156) zum Sichern des zweiten Armabschnitts (140) an Außenflächen des ersten Armabschnitts (130) und des zweiten Armabschnitts (140), die einander gegenüberliegen, angeordnet ist, wobei der Hauptkörper (120) dort dazwischen angeordnet ist.

15. Hämostatische Vorrichtung (100, 100A, 100B) nach einem der Ansprüche 9 bis 14,
wobei der zweite Armabschnitt (140) einen geneigten Abschnitt (141) und einen zweiten Armendabschnitt (143) aufweist, der kontinuierlich mit dem geneigten Abschnitt (141) ausgebildet ist und einen Endabschnitt des zweiten Armabschnitts (140) bildet, und
eine Breite des geneigten Abschnitts (141) größer ist als eine Breite des zweiten Armendabschnitts (140).

16. Hämostatische Vorrichtung (100, 100A, 100B) nach einem der Ansprüche 9 bis 15,
wobei ein Injektionsabschnitt (191) zum Injizieren eines Fluids an dem aufblasbaren Element (160) vorgesehen ist, und ein Polsterungselement (192), das einen aufblasbaren Raum aufweist, zwischen dem Injektionsabschnitt (191) und dem aufblasbaren Element (160) angeordnet ist.

## Revendications

1. Dispositif hémostatique (100, 100A, 100B) comprenant :
un élément de recouvrement (110) configuré pour être disposé pour recouvrir un site (t1) où un saignement doit être arrêté sur une main (H) d'un patient ;
un élément de fixation (151, 152, 153, 154, 155, 156) configuré pour fixer l'élément de recouvrement (110) dans un état où l'élément de recouvrement (110) recouvre le site (t1) où un saignement doit être arrêté ; et
un élément gonflable (160) relié à l'élément de recouvrement (110) et configuré pour être gonflé par injection d'un fluide,
dans lequel l'élément de recouvrement (110) comporte un corps principal (120) auquel l'élément gonflable (160) est relié, une première partie de bras (130) faisant saillie du corps principal (120), et une deuxième partie de bras (140) faisant saillie du corps principal (120) tout en formant un angle obtus (θ1) avec une direction longitudinale de la première partie de bras (130),
la première partie de bras (130) présente une partie convexe (133a) faisant saillie dans une direction de largeur de la première partie de bras (130) au niveau d'une partie d'extrémité (133) de la première partie de bras (130), et
la deuxième partie de bras (140) est plus longue que la première partie de bras (130) et est configurée pour être fixée à la partie convexe (133a) et fixée à la deuxième partie de bras (140) en passant entre les doigts du patient dans un état où la deuxième partie de bras (140) est enroulée autour d'un membre du patient,
**caractérisé en ce que**
l'élément gonflable (160) présente une première partie gonflable (170) et un élément auxiliaire déformable (180) présentant une forme extérieure plus petite qu'une forme extérieure de la première partie gonflable (170), et
l'élément auxiliaire (180) est disposé pour chevaucher la première partie gonflable (170) sur un côté distal du corps principal (120).

2. Dispositif hémostatique (100, 100A, 100B) selon la revendication 1,
dans lequel le corps principal (120) présente une première zone (121a, 121b) et une deuxième zone (122) dans laquelle l'élément gonflable (160) est disposé différemment de la première zone (121a, 121b),
la première zone (121a, 121b) est disposée entre la deuxième zone (122) et la première partie de bras (130) et entre la deuxième zone (122) et la deuxième partie de bras (140), et
la deuxième zone (122) présente un élément de support (125) présentant une rigidité plus élevée qu'une rigidité de la première zone (121a, 121b).

3. Dispositif hémostatique (100, 100A, 100B) selon la revendication 2, dans lequel l'élément de support (125) présente une partie courbée (125a) formée sur un côté distal du corps principal (120).

4. Dispositif hémostatique (100, 100A, 100B) selon l'une quelconque des revendications 1 à 3,
dans lequel l'élément auxiliaire (180) est une deuxième partie gonflable (180) configurée pour être gonflée par injection d'un fluide, et
une lumière (181) de la deuxième partie gonflable (180) communique avec une lumière (171) de la première partie gonflable (170).

5. Dispositif hémostatique (100, 100A, 100B) selon l'une quelconque des revendications 1 à 4,
dans lequel l'élément auxiliaire (180) est disposé pour présenter une symétrie bilatérale par rapport à une ligne centrale de la première partie gonflable (170), et
l'élément de fixation (151, 152, 153, 154, 155, 156) pour fixer la deuxième partie de bras (140) est disposé sur les surfaces extérieures de la première partie de bras (130) et la deuxième partie de bras (140) opposée l'une à l'autre avec le corps principal (120) interposé entre celles-ci.

6. Dispositif hémostatique (100, 100A, 100B) selon l'une quelconque des revendications 1 à 5, dans lequel la partie convexe (133a) fait saillie dans une direction croisant une direction d'extension de la première partie de bras (130) en direction d'un côté distal du corps principal (120).

7. Dispositif hémostatique (100, 100A, 100B) selon l'une quelconque des revendications 1 à 6,
dans lequel la deuxième partie de bras (140) présente une partie inclinée (141) et une deuxième partie d'extrémité de bras (143) qui est formée en continu avec la partie inclinée (141) et forme une partie d'extrémité de la deuxième partie de bras (140), et
une largeur de la partie inclinée (141) est plus grande qu'une largeur de la deuxième partie d'extrémité de bras (143).

8. Dispositif hémostatique (100, 100A, 100B) selon l'une quelconque des revendications 1 à 7,
dans lequel une partie d'injection (191) pour injecter un fluide est prévue sur l'élément gonflable (160), et
un élément d'amortissement (192) présentant un espace gonflable est disposé entre la partie d'injection (191) et l'élément gonflable (160).

9. Dispositif hémostatique (100, 100A, 100B) comprenant :
un élément de recouvrement (110) configuré pour être disposé pour recouvrir un site (t1) où un saignement doit être arrêté sur une main (H) d'un patient ;
un élément de fixation (151, 152, 153, 154, 155, 156) configuré pour fixer l'élément de recouvrement (110) dans un état où l'élément de recouvrement (110) recouvre le site (t1) où un saignement doit être arrêté ; et
un élément gonflable (160) relié à l'élément de recouvrement (110) et configuré pour être gonflé par injection d'un fluide,
dans lequel l'élément de recouvrement (110) comporte un corps principal (120) auquel l'élément gonflable (160) est relié, une première partie de bras (130) faisant saillie du corps principal (120), et une deuxième partie de bras (140) faisant saillie du corps principal (120) tout en formant un angle obtus avec une direction longitudinale de la première partie de bras (130),
la première partie de bras (130) et la deuxième partie de bras (140) de l'élément de recouvrement (110) sont configurées pour pouvoir être reliées par l'élément de fixation (151, 152, 153, 154, 155, 156) dans un état où l'élément de recouvrement (110) est enroulé autour d'un membre du patient, et
**caractérisé en ce que** l'élément gonflable (160) présente une première partie gonflable (170) et un élément auxiliaire déformable (180) qui présente une forme extérieure plus petite qu'une forme extérieure de la première partie gonflable (170) et est sollicitée en direction d'un côté d'extrémité de la première partie gonflable (170) et **en ce que**
l'élément auxiliaire (180) est disposé entre la première partie de bras (130) et la deuxième partie de bras (140).

10. Dispositif hémostatique (100, 100A, 100B) selon la revendication 9,
dans lequel le corps principal (120) présente une première zone (121a, 121b) et une deuxième zone (122) dans laquelle l'élément gonflable (160) est disposé différemment de la première zone (121a, 121b),
la première zone (121a, 121b) est disposée entre la deuxième zone (122) et la première partie de bras (130) et entre la deuxième zone (122) et la deuxième partie de bras (140), et
la deuxième zone (122) présente un élément de support (125) présentant une rigidité plus élevée qu'une rigidité de la première zone (121a, 121b).

11. Dispositif hémostatique (100, 100A, 100B) selon la revendication 10, dans lequel l'élément de support (125) présente une partie courbée (125a) formée sur un côté distal du corps principal (120).

12. Dispositif hémostatique (100, 100A, 100B) selon l'une quelconque des revendications 9 à 11,
dans lequel l'élément auxiliaire (180) est une deuxième partie gonflable (180) configurée pour être gonflée par injection d'un fluide, et
une lumière (181) de la deuxième partie gonflable (180) communique avec une lumière (171) de la première partie gonflable (170).

13. Dispositif hémostatique (100, 100A, 100B) selon l'une quelconque des revendications 9 à 12, dans lequel la deuxième partie de bras (140) est plus longue que la première partie de bras (130) et est configurée pour être fixée à la deuxième partie de bras (140) en passant entre les doigts du patient dans un état où la deuxième partie de bras (140) est enroulée autour du membre du patient.

14. Dispositif hémostatique (100, 100A, 100B) selon l'une quelconque des revendications 9 à 13,
dans lequel l'élément auxiliaire (180) est disposé pour présenter une symétrie bilatérale par rapport à une ligne centrale de la première partie gonflable (170), et
l'élément de fixation (151, 152, 153, 154, 155, 156) pour fixer la deuxième partie de bras (140) est disposé sur les surfaces extérieures de la première partie de bras (130) et la deuxième partie de bras (140) opposées l'une à l'autre avec le corps principal (120) interposé entre celles-ci.

15. Dispositif hémostatique (100, 100A, 100B) selon l'une quelconque des revendications 9 à 14,
dans lequel la deuxième partie de bras (140) présente une partie inclinée (141) et une deuxième partie d'extrémité de bras (143) qui est formée en continu avec la partie inclinée (141) et forme une partie d'extrémité de la deuxième partie de bras (140), et
une largeur de la partie inclinée (141) est plus grande qu'une largeur de la deuxième partie d'extrémité de bras (140).

16. Dispositif hémostatique (100, 100A, 100B) selon l'une quelconque des revendications 9 à 15,
dans lequel une partie d'injection (191) pour injecter un fluide est prévue sur l'élément gonflable (160), et
un élément d'amortissement (192) présentant un espace gonflable est disposé entre la partie d'injection (191) et l'élément gonflable (160).
